# EUROPEAN PATENT SPECIFICATION

(11) **EP 2 540 835 B1**
(45) Date of publication and mention of the grant of the patent: **09.05.2018**
(21) Application number: 11747340.5
(22) Date of filing: 22.02.2011
(51) Int. Cl.: C12P 7/62, C12N 9/88, C12N 15/09

(54) **PROCESS FOR PRODUCTION OF POLY(3-HYDROXYBUTYRATE-CO-3-HYDROXYHEXANOATE) USING A GENETICALLY MODIFIED CUPRIAVIDUS NECATOR HAVING AN ENOYL-CoA HYDRATASE GENE INTRODUCED THEREIN**
VERFAHREN ZUR HERSTELLUNG VON POLY(3-HYDROXYBUTYRAT-CO-3-HYDROXYHEXANOAT) MITTELS GENETISCH MODIFIZIERTER CUPRIAVIDUS NECATOR MIT EINEM DARIN EINGEFÜHRTEN ENOYL-CoA-HYDRATASE-GEN
PROCÉDÉ DE PRODUCTION DE POLY(3-HYDROXYBUTYRATE-CO-3-HYDROXYHEXANOATE) UTILISANT UN CUPRIAVIDUS NECATOR GÉNÉTIQUEMENT MODIFIÉ AYANT UN GÈNE D'ÉNOYL-CoA HYDRATASE INTRODUIT EN LUI

(30) Priority: 26.02.2010 JP 2010043017
(43) Date of publication of application: 02.01.2013
(73) Proprietor: Tokyo Institute of Technology, Tokyo 152-8550 (JP); Kaneka Corporation, Osaka (JP)
(72) Inventor: FUKUI, Toshiaki, Yokohama-shi Kanagawa 226-8503 (JP); ORITA, Izumi, Yokohama-shi Kanagawa 226-8503 (JP); MIFUNE, Jun, Yokohama-shi Kanagawa 226-8503 (JP); KAWASHIMA, Yui, Yokohama-shi Kanagawa 226-8503 (JP)
(74) Representative: Awapatent AB
(86) International application number: PCT/JP2011/053861
(87) International publication number: WO 2011/105379

(56) References cited:
- WO-A1-2008/090873
- JP-A- 2002 521 060
- MIFUNE ET AL: "Targeted engineering of Cupriavidus necator chromosome for biosynthesis of poly(3-hydroxybutyrate-co-3-hydroxyhexanoa te) from vegetable oil", CANADIAN JOURNAL OF CHEMISTRY, vol. 86, 2008, pages 621-627, XP008163624,
- TSUGE ET AL: "Combination of N149S and D171G mutations in Aeromonas caviae polyhydroxyalkanoate synthase and impact on polyhydroxyalkanoate biosynthesis", FEMS MICROBIOLOGY LETTERS, vol. 277, 2007, pages 217-222, XP002702996,
- HU ET AL: "Site-directed mutagenesis of Aeromonas hydrophila enoyl Coenzyme A hydratase enhancing 3-hydroxyhexanoate fractions of poly(3-hydroxybutyrate-co-3-hydroxyhexanoa te)", CURRENT MICROBIOLOGY, vol. 55, 2007, pages 20-24, XP002702997,
- FUKUI ET AL: "Microbial synthesis of poly((R)-3-hydroxybutyrate-co-3-hydroxypro pionate) from unrelated carbon sources by engineered Cupriavidus necator", BIOMACROMOLECULES, vol. 10, 2009, pages 700-706, XP002702995,
- MIFUNE ET AL: "Engineering of pha operon on Cupriavidus necator chromosome for efficient biosynthesis of poly(3-hydroxybutyrate-co-3-hydroxyhexanoa te) from vegetable oil", POLYMER DEGRADATION AND STABILITY, vol. 95, 1 March 2010 (2010-03-01), pages 1305-1312, XP027122897,
- JIAN ET AL: "Metabolic engineering for microbial production of polyhydroxyalkanoates consisting of high 3-hydroxyhexanoate content by recombinant Aeromonas hydrophila", BIORESOURCE TECHNOLOGY, vol. 101, 16 March 2010 (2010-03-16), pages 6096-6102, XP027018554,
- QIN, L. F. ET AL.: 'Biosynthesis of polyhydroxyalkanoate copolyesters by Aeromonas hydrophila mutant expressing a low-substrate- specificity PHA synthase PhaC2ps' BIOCHEMICAL ENGINEERING JOURNAL vol. 37, 15 November 2007, pages 144 - 150, XP022300244
- TOSHIAKI FUKUI: 'Microbial synthesis of optically active polyesters and 3-hydroxyalkanoic acids' GEKKAN FINE CHEMICAL vol. 38, no. 5, 15 April 2009, pages 32 - 42, XP008166919
- SATO ET AL: "Poly[(R)-3-hydroxybutyrate] formation in Escherichia coli from glucose through an enoyl-CoA hydratase-mediated pathway", JOURNAL OF BIOSCIENCEE AND BIOENGINEERING, vol. 103, 2007, pages 38-44, XP005885248,
- FUKUI ET AL: "Cloning and analysis of the poly(3-hydroxybutyrate-co-3-hydroxyhexanoa te) biosynthesis genes of Aeromonas caviae", JOURNAL OF BACTERIOLOGY, vol. 179, 1997, pages 4821-4830, XP002113257,

## Description

### Field of the Invention

The present invention relates to a method for microbially producing poly(3-hydroxybutyrate-co-3-hydroxyhexanoate), one of the copolyesters that can be microbially biodegradable and excellently biocompatible, using a vegetable oil as a basic raw material.

### Background Art

While petrochemical plastics, an essential material in modern society, are inexpensive and easily processed, they are posing a disposal problem due to their persistent nature. Thus, polyhydroxyalkanoates (PHAs) intracellularly accumulated as an energy source by various microorganisms are expected as a plastic material having a small environmental burden that uses biomass in stead of petroleum as a raw material and that is biodegradable. Poly(3-hydroxybutyric acid) (hereinafter referred to as "P(3HB)") is a representative PHA that is biosynthesized by various microorganisms. However, because of its physical properties of being hard and brittle, P(3HB) may be hard to be put into practical use.

On the other hand, poly(3-hydroxybutyrate-co-(R)-3-hydroxyhexanoate) copolymer (hereinafter referred to as "P(3HB-co-3HHx)") synthesized from a vegetable oil by Aeromonas caviae (A. caviae) exhibits an excellent property of being flexible. Furthermore, since this copolymer may exhibit a wide variety of physical properties that may be applicable to hard polymers to soft polymers depending on the 3HHx composition, its application into a variety of uses may be expected (Non-patent document 1).

So far, A. caviae has been known to use a vegetable oil as a raw material to synthesize P(3HB-co-3HHx) in which the 3HHx (hydroxyhexanoic acid) fraction is 9-13 mol%. However, its intracellular content is as low as about 6-12% by weight, which is difficult to be put into practical production (Patent Document 1, Patent Document 2, Non-patent document 1). Thus, the present inventors have constructed a recombinant strain of a hydrogen-oxidizaing bacterium Cupriavidus necator (C. necator) that accumulates P(3HB-co-3HHx) with a high content (70-80% by weight) from vegetable oils by introducing phaC_{AC}, a PHA synthase (polymerase) derived from A. caviae, into the PHB-4 strain, a PHA accumulation-deficient strain of C. necator that efficiently produces P(3HB). In this case, however, there were problems that the 3HHx fraction was as low as 4-5 mol% and the PHB-4 strain had a reduced growth ability compared to the wild type strain H16 (Patent Document 3, Non-patent document 2). Since the 3HHx fraction may preferably be 7-15 mol% in order to make polymers having a feasible flexibility, a method of constructing a recombinant strain that can efficiently accumulate P(3HB-co-3HHx) with a high 3HHx fraction and that has a high growth ability has been sought after. Though the present inventors constructed a strain that exhibits a high growth ability and a high PHA producing ability by introducing phaC_{Ac} into the chromosome of C. necator strain H16, a decrease in the 3HHx fraction was observed (Non-patent document 3). There is also disclosed a method in which phaC_{NSDG} gene encoding a mutant enzyme of A. caviae-derived PHA synthase was introduced into the chromosome of a mutant strain of C. necator in which expression of β-ketothiolase gene and acetoacetyl-CoA reductase, enzymes that supply 3HB-CoA monomers, was weakened (Patent Document 4).

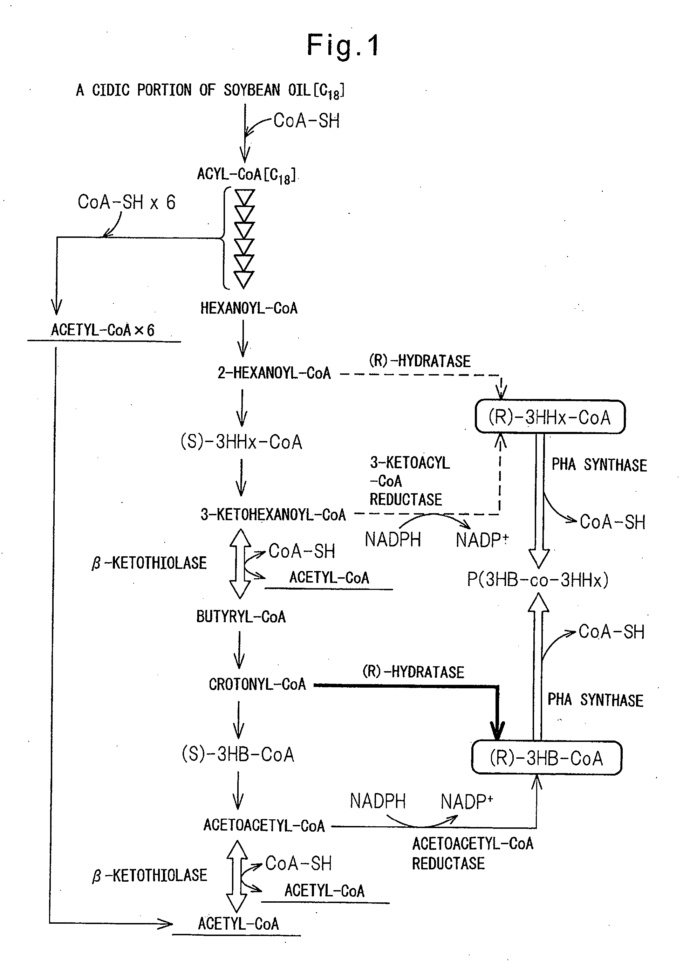

On the other hand, the present inventors demonstrated, based on the analysis of PHA biosynthesis by A. caviae, that phaJ_{AC}, a gene located immediately downstream to PHA synthase, encodes (R)-form-specific enoyl-CoA hydratase (hereinafter referred to simply as "R-hydratase") that converts enoyl-CoA, an intermediate in the fatty acid β-oxidation system, to (R)-3HA-CoA (Fig. 1) (Patent Document 3, Non-patent document 4). The present inventors demonstrated that by introducing both of phaJ_{AC} and phaC_{Ac} into Escherichia coli (E. coli), an ability of biosynthesizing P(3HB-co-3HHx) using fatty acid as carbon source can be conferred to E. coli having no potential ability of synthesizing PHA (Non-patent document 5). Since the discovery of R-hydratase in A. caviae, similar R-hydratases and their genes have been found in various microorganisms, and it has been shown that by introducing it together with a suitable PHA polymerase gene into E. coli having no PHA synthetic ability, it can be used in the microbial synthesis of copolymer PHA consisting of C4-C6 hydroxyalkanoic acids or copolymer PHA consisting of middle chain-length C6-C12 3-hydroxyalkanoic acids (Non-patent documents 6, 7 and 8, Patent Document 5). There were problems, however, that since E. coli has a low growth ability when fatty acids are used as the carbon sources, PHA productivity in these examples is low, and E. coli cannot .utilize inexpensive vegetable oils as the carbon sources.
Journal of Bioscience and Bioengineering, Vol. 103, No. 1, 38-44, 2007 describes a new metabolic pathway for the synthesis of poly[(R)-3-hydroxybutyrate] [P(3HB)] which was constructed in a recombinant Escherichia coli strain, where the reactions were catalyzed by two substrate-specific enoyl-CoA hydratases.
Polymer Degradation and Stability 95; March 2010, pp. 1305-1312, describes engineering of pha operon on C. *necator* chromosome for biosynthesis of P(3HB-co-3HHc) copolyester from soybean oil.
Biochem Eng J; 2007, pp. 144-150 relates to biosynthesis of p(3HB-co-3HHx) by coexpressing (R)-specific enoyl-CoA hydratase and PHA synthase PhaC2_{Ps} in *Aeromonas hydrophila CQ4.*

On the other hand, in the construction of recombinant strains derived from the above C. necator strain PHB⁻4, various recombinant strains in which phaJ_{AC} was introduced together with phaC_{AC} were constructed, and PHA biosynthesis using octanoic acid as the carbon source was investigated. However, no clear correlation was observed between the PHA accumulation rate or 3HHx fraction and phaJ_{AC} introduction in these strains (Patent Document 3, Non-patent documents 9 and 10). Thus, the effect of introducing the R-hydratase gene in the biosynthesis of P(3HB-co-3HHx) using a C. necator strain has not been clarified. There are no reports, either, that C. necator has R-hydratase.

### Prior Art Publications

### Patent Documents

Patent Document 1: Japanese Unexamined Patent Publication (Kokai) No. 5-93049
Patent Document 2: Japanese Unexamined Patent Publication (Kokai) No. 7-265065
Patent Document 3: Japanese Patent No. 3062459
Patent Document 4: Japanese Unexamined Patent Publication (Kokai) No. 2008-29218
Patent Document 5: Japanese Unexamined Patent Publication (Kokai) No. 2004-321167

### Non-patent documents

Non-patent document 1: Doi et al., Macromolecules, 28:4822-4828 (1995)
Non-patent document 2: Fukui et al., Appl. Microbiol. Biotechnol., 49:333-336 (1998)
Non-patent document 3: Mifune et al., Can. J. Chem., 86:621-627 (2008)
Non-patent document 4: Fukui et al., J. Bacteriol. 180:667-673 (1998)
Non-patent document 5: Fukui et al., FEMS Microbiol. Lett., 170:69-75 (1999)
Non-patent document 6: Reiser et al., Appl. Microbiol. Biotechnol., 53:209-218 (2000)
Non-patent document 7: Tsuge et al., FEMS Microbiol. Lett., 184:193-198 (1999)
Non-patent document 8: Park et al., J. Bacteriol. 185:5391-5397 (2003)
Non-patent document 9: Fukui et al., J. Bacteriol. 179:4821-4830 (1997)
Non-patent document 10: Kichise et al., Int. J. Biol. Macromolecules 25:69-77 (1999)

### Summary of the Invention

### Means to Solve the Problems

The present inventors have found that by introducing a gene encoding R-hydratase that converts intermediates in the fatty acid β-oxidation system to (R)-3-hydroxyacyl-CoA [R-3HA-CoA] monomers into a recombinant C. necator strain to which a P(3HB-co-3HHx)-producing ability has been conferred, a microorganism that produces P(3HB-co-3HHx) using a vegetable oil as a basic raw material can be constructed, and thereby have completed the present invention.

In accordance with the present invention, there are provided (i) a method of producing P(3HB-co-3HHx) having a high 3HHx fraction at a high accumulation rate using only a vegetable oil as the raw material by introducing a R-hydratase gene derived from a known A. caviae into the chromosome of a recombinant C. necator strain to which a P(3HB-co-3HHx)-producing ability has been conferred, and (ii) a method of producing P(3HB-co-3HHx) having a high 3HHx fraction at a high content using only a vegetable oil as the raw material by introducing a R-hydratase gene newly identified from a hydrogen bacterium C. necator into a recombinant C. necator strain to which a P(3HB-co-3HHx)-producing ability has been conferred.

More specifically, in accordance with the present invention, there is provided a method of producing P(3HB-co-3HHx), which method comprises transforming, by homologous recombination, the R- form-specific enoyl CoA hydratase gene into the chromosome of a recombinant C. necator strain to which a P(3HB-co-3HHx)-producing ability has been conferred, or transforming by introducing into said strain an autonomously replicating vector having said gene integrated therein, and growing the transformant in a medium containing a vegetable oil as a carbon source, wherein the fraction of 3HHx is 5-20 mol% and the content of P(3HB-co-3HHx) in the transformant is 50-90% by weight.

The C. necator strain that can be used in the method of the present invention may be, but not limited to, a NSDG strain, a NSDGΔA strain, or a MF01 strain.

In one embodiment of the present invention, the R-hydratase gene for use in the production method of the present invention:
is derived from an A. caviae strain,, and comprises
(a) a nucleic acid comprising the base sequence set forth in SEQ ID NO: 1, or
(b) a nucleic acid that hybridizes to a nucleic acid comprising the base sequence set forth in SEQ ID NO: 1 under a stringent condition and that encodes a protein having an activity of converting a fatty acid β-oxidation system intermediate to (R)-3-hydroxyacyl-CoA.

In one embodiment of the present invention, the R form-specific enoyl CoA hydratase gene:
is derived from a C. necator strain, and comprises
(a) a nucleic acid comprising the base sequence set forth in SEQ ID NO: 2, or
(b) a nucleic acid that hybridizes to a nucleic acid comprising the base sequence set forth in SEQ ID NO: 2 under a stringent condition and that encodes a protein having an activity of converting a fatty acid β-oxidation system intermediate to (R)-3-hydroxyacyl-CoA. Alternatively, it comprises
   (a) a nucleic acid comprising the base sequence set forth in SEQ ID NO: 3, or
   (b) a nucleic acid that hybridizes to a nucleic acid comprising the base sequence set forth in SEQ ID NO: 3 under a stringent condition and that encodes a protein having an activity of converting a fatty acid β-oxidation system intermediate to (R)-3-hydroxyacyl-CoA.

In one embodiment of the present invention, the R form-specific enoyl CoA hydratase gene:
- is derived from an A. caviae strain, and comprises
   (a) a nucleic acid comprising the base sequence set forth in SEQ ID NO: 1, or
   (b) a nucleic acid that hybridizes to a nucleic acid comprising the base sequence set forth in SEQ ID NO: 1 under a stringent condition and that encodes a protein having an activity of converting a fatty acid β-oxidation system intermediate to (R)-3-hydroxyacyl-CoA; and
- is derived from a C. necator strain, and comprises
   (a) a nucleic acid comprising the base sequence set forth in SEQ ID NO: 2, or
   (b) a nucleic acid that hybridizes to a nucleic acid comprising the base sequence set forth in SEQ ID NO: 2 under a stringent condition and that encodes a protein having an activity of converting a fatty acid β-oxidation system intermediate to (R)-3-hydroxyacyl-CoA.

In one embodiment of the present invention, the R form-specific enoyl CoA hydratase gene:
- is derived from an A. caviae strain, and comprises
   (a) a nucleic acid comprising the base sequence set forth in SEQ ID NO: 1, or
   (b) a nucleic acid that hybridizes to a nucleic acid comprising the base sequence set forth in SEQ ID NO: 1 under a stringent condition and that encodes a protein having an activity of converting a fatty acid β-oxidation system intermediate to (R)-3-hydroxyacyl-CoA; and
- is derived from a C. necator strain, and comprises
   (a) a nucleic acid comprising the base sequence set forth in SEQ ID NO: 3, or
   (b) a nucleic acid that hybridizes to a nucleic acid comprising the base sequence set forth in SEQ ID NO: 3 under a stringent condition and that encodes a protein having an activity of converting a fatty acid β-oxidation system intermediate to (R)-3-hydroxyacyl-CoA.

### Effects of the Invention

By introducing R-hydratase into a P(3HB-co-3HHx)-producing microorganism, a strain can be produced that produces P(3HB-co-3HHx) having a high 3HHx fraction while maintaining a high growth ability and a high PHA productivity.

### Brief Explanation of the Drawings

[Fig. 1]
   A biosynthetic pathway for P(3HB-co-3HHx) in A. caviae.
[Fig. 2]
   The name of the recombinant C. necator strain constructed and the constitution of related genes on the chromosome.
[Fig. 3]
   The positions of genes in the recombinant C. necator strain.
[Fig. 4]
   The result of investigating the stereoselectivity of PhaJ1, PhaJ2 and PhaJ3 by the conjugation of the enoyl-CoA hydratase-mediated hydration reaction and the PHA granules-mediated polymerization reaction. (A) PhaJ1, (B) PhaJ2, and (C) PhaJ3; dark square: purified enzyme added, open triangle: no purified enzyme added.

### Mode for Carrying Out the Invention

For the purpose of explaining the present invention, preferred embodiments will be described in detail below.

As described above, the present invention provides a method of producing poly(3-hydroxybutyrate-co-3-hydroxyhexanoate) [P(3HB-co-3HHx)], which method comprises transforming, by homologous recombination, the (R)-form-specific enoyl-CoA hydratase [R-hydratase] gene into the chromosome of a recombinant Cupriavidus necator [C. necator] strain to which a P(3HB-co-3HHx)-producing ability has been conferred, or transforming by introducing into said strain an autonomously replicating vector having said gene integrated therein, and growing the transformant in a medium containing a vegetable oil as a carbon source, wherein the fraction of 3-hydroxyhexanoate (3HHx) is 5-20 mol% and the accumulation rate of P(3HB-co-3HHx) in the transformant is 50-90% by weight.

### (1) Recombinant C. necator strain

As a microorganism for use in the production method of the present invention, a recombinant C. necator strain to which a P(3HB-co-3HHx)-producing ability has been conferred may be preferred. As used herein the term "a recombinant C. necator strain to which a P(3HB-co-3HHx)-producing ability has been conferred" refers to, for the purpose of biosynthesizing P(3HB-co-3HHx), a microbial strain in which a broad substrate-specific PHA synthase (for example, the gene of A. caviae-derived PHA synthase or its mutant enzyme) capable of using 3HHx-CoA having 6 carbons as a substrate has been introduced into a C. necator strain in which PHA synthase inherently present in the native strain has been deleted by mutation or gene destruction, or a microbial strain in which the PHA synthase gene inherently present in the native C. necator strain has been replaced with the gene of a broad substrate-specific PHA synthase or its mutant enzyme, characterized in that it has an ability of synthesizing P(3HB-co-3HHx) which the wild type C. necator strain cannot biosynthesize due to the effect of the broad substrate-specific PHA synthase. Such a gene manipulation can be easily carried out by a common gene engineering method. As a recombinant C. necator strain to which the above P(3HB-co-3HHx)-producing ability has been conferred, there can be mentioned, but not limited to, a NSDG strain, a NSDGΔ strain, a MF01 strain etc. As used herein the term "a NSDG strain" refers to a transformant into which phaC_{NSDG}, the gene of a mutant PHA synthase enzyme, has been introduced into the chromosome of the H16 strain, one of hydrogen bacterium C. necator. "phaC_{NSDG}" refers to a phaC gene encoding PHA synthesizing enzyme in C. necator and to a mutant gene in which asparagine (N) at position 149 in the amino acid sequence (SEQ ID NO: 4) of phaC is replaced with serine (S) and aspartic acid (D) at position 171 is replaced with glycine (G). Also "a NSDGΔA strain" refers to a transformant in which a β-ketothiolase enzyme phaA_{Cn} in the above NSDG strain is deleted. On the other hand "a MF01 strain" refers to a transformant in which phaA_{Cn} of the above NSDG strain is replaced with a broad substrate-specific β-ketothiolase gene bktB_{Cn}. The above three H16 mutants can be constructed using a common gene engineering method based on the sequence information of the gene encoding the PHA synthase enzyme of C. necator (see, for example, Patent Document 4).

### (2) Cloning of R form-specific enoyl CoA hydratase ("R-hydratase") gene

"R-hydratase" for use in the production method of the present invention refers to an enzyme that converts a fatty acid β-oxidation system intermediate (for example enoyl-CoA) into a polyhydroxyalkanoic acid (PHA) monomer (R)-3-hydroxyacyl-CoA [R-3HA-CoA] (for example, (R)-3-hydroxybutyric acid [(R)-3HB] and (R)-3-hydroxyhexanoic acid [(R)-3HHx]). A nucleic acid encoding R-hydratase for use in the production method of the present invention may comprise a single stranded or double stranded DNA and a RNA complement thereof. DNA may comprise, for example, naturally occurring DNA, recombinant DNA, chemically synthesized DNA, PCR-amplified DNA, and combinations thereof. As a nucleic acid for use in the present invention, DNA may be preferred. As is well known, codons have degeneracy, and thus one amino acid may be encoded by a plurality of base sequences. In the case of base sequences of nucleic acids encoding R-hydratase, a nucleic acid having any of those base sequences may be encompassed by the present invention.

In the production method of the present invention, as a R-hydratase gene, a nucleotide 4475-4879 ("ORF3") in the base sequence (GenBank Accession No. D88825) of a gene (phaJ_{Ac}) encoding R-hydratase derived from an A. caviae strain can be used. As used herein, the base sequence of said gene is listed as SEQ ID NO: 1 in the Sequence Listing.

Also as used herein, as a R-hydratase gene, there can be used base sequences of the gene encoding R-hydratase derived from C. necator: (i) a nucleotide 1167077-1167553 ("H16_A1070") in GenBank Accession No. AM260479, corresponding to "phaJ1" in the present invention, and (ii) a nucleotide 454107-454562 ("H16_B0397") in GenBank Accession No. AM260480, corresponding to "phaJ2" in the present invention. As used herein, the base sequence of the gene of the above (i) is listed as SEQ ID NO: 2, and that of the above (ii) as SEQ ID NO: 3 in the Sequence Listing.

As one embodiment of the present invention, a gene encoding R-hydratase derived from an A. caviae strain comprises
(a) a nucleic acid comprising the base sequence set forth in SEQ ID NO: 1, or
(b) a nucleic acid that hybridizes to a nucleic acid comprising the base sequence set forth in SEQ ID NO: 1 under a stringent condition and that encodes a protein having an activity of converting a fatty acid β-oxidation system intermediate to (R)-3-hydroxyacyl-CoA.

As one embodiment of the present invention, a gene encoding R-hydratase derived from an A. caviae strain comprises
(a) a nucleic acid consisting of the base sequence set forth in SEQ ID NO: 1, or
(b) a nucleic acid that hybridizes to a nucleic acid consisting of the base sequence set forth in SEQ ID NO: 1 under a stringent condition and that encodes a protein having an activity of converting a fatty acid β-oxidation system intermediate to (R)-3-hydroxyacyl-CoA.

As one embodiment of the present invention, a gene encoding R-hydratase derived from a C. necator strain comprises
(a) a nucleic acid comprising the base sequence set forth in SEQ ID NO: 2, or
(b) a nucleic acid that hybridizes to a nucleic acid comprising the base sequence set forth in SEQ ID NO: 2 under a stringent condition and that encodes a protein having an activity of converting a fatty acid β-oxidation system intermediate to (R)-3-hydroxyacyl-CoA, alternatively
(a) a nucleic acid comprising the base sequence set forth in SEQ ID NO: 3, or
(b) a nucleic acid that hybridizes to a nucleic acid comprising the base sequence set forth in SEQ ID NO: 3 under a stringent condition and that encodes a protein having an activity of converting a fatty acid β-oxidation system intermediate to (R)-3-hydroxyacyl-CoA.

As a preferred embodiment of the present invention, a gene encoding R-hydratase derived from a C. necator strain comprises
(a) a nucleic acid consisting of the base sequence set forth in SEQ ID NO: 2, or
(b) a nucleic acid that hybridizes to a nucleic acid consisting of the base sequence set forth in SEQ ID NO: 2 under a stringent condition and that encodes a protein having an activity of converting a fatty acid β-oxidation system intermediate to (R)-3-hydroxyacyl-CoA, alternatively
(a) a nucleic acid consisting of the base sequence set forth in SEQ ID NO: 3, or
(b) a nucleic acid that hybridizes to a nucleic acid consisting of the base sequence set forth in SEQ ID NO: 3 under a stringent condition and that encodes a protein having an activity of converting a fatty acid β-oxidation system intermediate to (R)-3-hydroxyacyl-CoA.

As one embodiment of the present invention, an R form-specific enoyl CoA hydratase gene:
- is derived from an A. caviae strain, and comprises
   (a) a nucleic acid comprising the base sequence set forth in SEQ ID NO: 1, or
   (b) a nucleic acid that hybridizes to a nucleic acid comprising the base sequence set forth in SEQ ID NO: 1 under a stringent condition and that encodes a protein having an activity of converting a fatty acid β-oxidation system intermediate to (R)-3-hydroxyacyl-CoA; and
- is derived from a C. necator strain, and comprises
   (c) a nucleic acid comprising the base sequence set forth in SEQ ID NO: 2, or
   (d) a nucleic acid that hybridizes to a nucleic acid comprising the base sequence set forth in SEQ ID NO: 2 under a stringent condition and that encodes a protein having an activity of converting a fatty acid β-oxidation system intermediate to (R)-3-hydroxyacyl-CoA. The above gene encoding R-hydratase connects a nucleic acid (a or b) and a nucleic acid (c or d) adjacently or nonadjacently, and the order of the two nucleic acids connected is not limited, and furthermore the insertion site in the chromosome is not limited provided that the insertion is carried out under the control of an appropriate promoter.

As a preferred embodiment of the present invention, an R form-specific enoyl CoA hydratase gene:
- is derived from an A. caviae strain, and comprises
   (a) a nucleic acid consisting of the base sequence set forth in SEQ ID NO: 1, or
   (b) a nucleic acid that hybridizes to a nucleic acid consisting of the base sequence set forth in SEQ ID NO: 1 under a stringent condition and that encodes a protein having an activity of converting a fatty acid β-oxidation system intermediate to (R)-3-hydroxyacyl-CoA; and
- is derived from a C. necator strain, and comprises
   (c) a nucleic acid consisting of the base sequence set forth in SEQ ID NO: 2, or
   (d) a nucleic acid that hybridizes to a nucleic acid consisting of the base sequence set forth in SEQ ID NO: 2 under a stringent condition and that encodes a protein having an activity of converting a fatty acid β-oxidation system intermediate to (R)-3-hydroxyacyl-CoA. The above gene encoding R-hydratase connects a nucleic acid (a or b) and a nucleic acid (c or d) adjacently or nonadjacently, and the order of the two nucleic acids connected is not limited, and furthermore the insertion site in the chromosome is not limited provided that the insertion is carried out under the control of an appropriate promoter.

As one embodiment of the present invention, an R form-specific enoyl CoA hydratase gene:
- is derived from an A. caviae strain, and comprises
   (a) a nucleic acid comprising the base sequence set forth in SEQ ID NO: 1, or
   (b) a nucleic acid that hybridizes to a nucleic acid comprising the base sequence set forth in SEQ ID NO: 1 under a stringent condition and that encodes a protein having an activity of converting a fatty acid β-oxidation system intermediate to (R)-3-hydroxyacyl-CoA; and
- is derived from a C. necator strain, and comprises
   (c) a nucleic acid comprising the base sequence set forth in SEQ ID NO: 3, or
   (d) a nucleic acid that hybridizes to a nucleic acid comprising the base sequence set forth in SEQ ID NO: 3 under a stringent condition and that encodes a protein having an activity of converting a fatty acid β-oxidation system intermediate to (R)-3-hydroxyacyl-CoA. The above gene encoding R-hydratase connects a nucleic acid (a or b) and a nucleic acid (c or d) adjacently or nonadjacently, and the order of the two nucleic acids connected is not limited, and furthermore the insertion site in the chromosome is not limited provided that the insertion is carried out under the control of an appropriate promoter.

As a preferred embodiment of the present invention, an R form-specific enoyl CoA hydratase gene:
- is derived from an A. caviae strain, and comprises
   (a) a nucleic acid consisting of the base sequence set forth in SEQ ID NO: 1, or
   (b) a nucleic acid that hybridizes to a nucleic acid consisting of the base sequence set forth in SEQ ID NO: 1 under a stringent condition and that encodes a protein having an activity of converting a fatty acid β-oxidation system intermediate to (R)-3-hydroxyacyl-CoA; and
- is derived from a C. necator strain, and comprises
   (c) a nucleic acid consisting of the base sequence set forth in SEQ ID NO: 3, or
   (d) a nucleic acid that hybridizes to a nucleic acid consisting of the base sequence set forth in SEQ ID NO: 3 under a stringent condition and that encodes a protein having an activity of converting a fatty acid β-oxidation system intermediate to (R)-3-hydroxyacyl-CoA. The above gene encoding R-hydratase connects a nucleic acid (a or b) and a nucleic acid (c or d) adjacently or nonadjacently, and the order of the two nucleic acids connected is not limited, and furthermore the insertion site in the chromosome is not limited provided that the insertion is carried out under the control of an appropriate promoter.

As described in Examples 10 and 16 below, the above R-hydratase gene may be amplified by designing synthetic nucleotides as primers (SEQ ID NOs: 17 and 18, SEQ ID NOs: 23 and 24, and SEQ ID NOs: 25 and 26, respectively) based on the base sequences of SEQ ID NOs: 1 to 3, and using the chromosome DNA of a C. necator strain, a chromosome DNA-containing plasmid etc. as the template. As techniques for amplifying nucleic acid, there can be used, but not limited to, reactions that require temperature cycles such as a polymerase chain reaction (PCR) (Saiki R.K. et al., Science 230:1350-1354 (1985)), a ligase chain reaction (LCR) (Wu D.Y. et al., Genomics 4:560-569 (1989)) and transcription-based amplification (Kwoh D.Y. et al., Proc. Natl. Acad. Sci. U.S.A. 86:1173-1177 (1989)), as well as constant-temperature reactions such as strand displacement amplification (SDA) (Walker G.T. et al., Proc. Natl. Acad. Sci. U.S.A. 89:392-396 (1992); Walker G.T. et al., Nuc. Acids Res. 20:1691-1696 (1992)), self-sustained sequence replication (3SR) (Guatelli J.C., Proc. Natl. Acad. Sci. U.S.A. 87:1874-1878 (1990)), and Qβ replicase system (Lizardi, P.M. et al., BioTechnology 6:1197-1202 (1988)). In the production method of the present invention, the PCR method may preferably be used.

As used herein the term "under a stringent condition" means hybridization is carried out under a medium or high stringent condition. Specifically, a medium stringent condition may be easily determined by a person skilled in the art based on the length of DNA. A basic condition is indicated in Sambrook, J. et al., Molecular Cloning: A Laboratory Manual (3rd edition), Cold Spring Harbor Laboratory, 7.42-7.45 (2001), and, with regard to nitrocellulose filter, the use of a prewash solution of 5×SSC, 0.5% SDS, 1.0 mM EDTA (pH 8.0), a hybridization condition at about 40-50°C with about 50% formamide and 2xSSC - 6×SSC (or another similar hybridization solution such as Stark's solution in about 50% formamide at 42°C), and a washing condition at about 60°C with 0.5×SSC, and 0.1% SDS may be included. A high stringent condition may also be easily determined by a person skilled in the art based on the length of DNA. Generally such a condition may include hybridization condition and/or washing at a higher temperature and/or a lower salt concentration than in the medium stringent condition, and for example involve a hybridization condition as described above at about 68°C with 0.2×SSC and 0.1% SDS. A person skilled in the art will recognize that the temperature and the salt concentration of the washing solution may be adjusted, as needed, depending on factors such as the length of the probe.

A homologous nucleic acid cloned using the nucleic acid amplification reaction or hybridization as described above may have an identity of at least 30% or more, preferably 50% or more, more preferably 70% or more, even more preferably 90% or more, and most preferably 95% or more with the base sequences set forth in SEQ ID NO: 1, SEQ ID NO: 2 and SEQ ID NO: 3 of the Sequence Listing.

The identity percent can be determined by visual inspection or by mathematical calculation. Alternatively, an identity percent between two nucleic acid sequences may be determined by comparing sequence information using a GAP computer program (GCG Wisconsin Package, version 10.3) described in Devereux et al., Nucl. Acids Res. 12:387 (1984) and available from the University of Wisconsin Genetics Computer Group (UWGCG).

As the most preferred embodiment of the present invention, R-hydratase derived from A. caviae is a nucleic acid consisting of the base sequence set forth in SEQ ID NO: 1 as, and R-hydratase derived from C. necator is a nucleic acid consisting of the base sequence set forth in SEQ ID NO: 2 or 3.

### (3) Construction of a gene replacement vector and generation of a recombinant microorganism

In accordance with the present invention, there can be provided a gene replacement vector in which a gene encoding R-hydratase has been integrated into a vector for homologous recombination or an expression vector in which said gene has been integrated into an autonomous replicating vector. As used herein, as a method for integrating a gene into a vector, there can be mentioned a method described in, for example, Sambrook, J. et al., Molecular Cloning: A Laboratory Manual (3rd edition), Cold Spring Harbor Laboratory, 1.1 (2001). Conveniently, a commercially available ligation kit (such as one manufactured by Toyobo) may be used.

A vector can conveniently be prepared by connecting the desired gene to a vector (such as plasmid DNA) for recombination commercially available in the technical field to which the present invention pertains using a standard method. As a vector for use in the method for producing a polyhydroxyalkanoate (PHA) copolymer of the present invention, for the purpose of replacing a microorganism-derived PHA synthase gene already integrated in the microbial chromosome with a foreign broad substrate-specific polyester polymerase gene in the microorganism, there may preferably be used, but not limited to, a vector for homologous recombination, pK18mobsacB (Schafer et al., Gene 145:69-73 (1994)), pJQ200 (Qandt, J. and Hynes, M.P., "Versatile suicide vectors which allow direct selection for gene replacement in gram negative bacteria," Gene (1993) 127:15-21). Alternatively, there can be illustrated, but not limited to, broad host-range vectors, pBBR1-MCS2 (GenBank Accession No. U23751), pJRD215 (M16198) (see Davision, J. et al., (1987) Gene 51:275-80), and pJB861 (U82000), pHRP311 (see Parales, R.E. and Harwood, C.S. (1993) Gene, 133:23-30) known to autonomously replicate in gram negative bacteria. As a plasmid for E. coli, there can be used pBAD24 (GenBank Accession No. X81837), pDONR201, pBluescript, pUC118, pUC18, pUC19, pBR322 and the like.

Also, a person skilled in the art could select, as appropriate, a restriction end so as to be compatible with a recombinant vector, and, furthermore, in order to express the desired protein, could select, as appropriate, a recombinant vector suitable for the host cells. By suitably arranging or introducing regions (as needed, an autonomous origin of replication, a transconjutation region, a selection marker such as a kanamycin-resistant gene, and the like) that serve to trigger homologous recombination between the gene for use in the present invention and the gene of the host cell of interest, such a vector has been or should be constructed so that the nucleic acid can be suitably recombined. The construction of vectors is described in Examples 10, 11, 13, and 16.

Generally, a transformant can be generated by integrating a recombinant vector into the host cell. As the host cell in this case, both prokaryotic cells (for example, E. coli (strain S17-1 etc.), Bacillus subtilis) and eukaryotic cells (mammal cells, yeast, insect cells, etc.) can be used. The introduction of a recombinant vector into the host cell (transformation) may be carried out using a known method. For example, in the case of bacteria (E. coli, Bacillus subtilis, etc.), there can be mentioned, ethyl, the method of Cohen et al. (Proc. Natl. Acad. Sci. U.S.A. 69:2110 (1972)), the protoplast method (Mol. Gen. Genet. 168:111 (1979)), the competent method (J. Mol. Biol. 56:209 (1971)), the calcium chloride method, the electroporation method and the like. Also, in the introduction of an expression vector into cells belonging to genus Ralstonia, genus Alkaligenes, genus Pseudomonas etc., the transconjutation method can be used (J. Bacteriol. 148:198 (1981)).

The transconjutation method simply utilizes the property of the cell of transferring a chromosome genome or a plasmid from one cell to another on contact of the cells with each other, and a means of permitting gene introduction by a series of steps, starting from the conjugation of a donor cell having introduced therein a self-transmissible plasmid harboring the DNA of interest and a receptor cell having no such plasmid, followed by the bridge formation in both cells, the replication and transfer of said plasmid, as well as the completion of DNA synthesis and cell separation.

As a host cell for use in the production method of the present invention, as described above, a recombinant C. necator strain to which the P(3HB-co-3HHx)-producing ability has been conferred may be preferred. More preferred is a NSDG strain, a NSDGΔA strain, or a MF01 strain. In Example 4 described below, a constructed gene replacement vector pK18msNSDG-AB was introduced into the above strain by transconjutation, and a recombinant strain in which the vector was introduced into the chromosome by single cross-over homologous recombination at the homologous region was isolated.

The position where the R-hydratase gene is introduced on the chromosome may not be specifically limited as long as it is located at a position where said gene may be expressed under the control of an existing control sequence on the chromosome. In one embodiment, a R-hydratase gene may be inserted at a region in the same transcription unit as the phaC_{NSDG} gene. More preferably, it may be inserted downstream to the phaC_{NSDG} gene.

### (4) Synthesis of a copolyester

Polyester synthesis may be carried out by culturing the above recombinant C. necator strain (for example, a H16C_{Ac} strain, a NSDG strain, a NSDGΔA strain, or a MF01 strain) to which the P(3HB-co-3HHx)-producing ability has been conferred in a given medium containing a vegetable oil so as to allow the production and accumulation of a copolyester in the cultured cells (for example, the cell mass) or in the culture (for example, culture medium), and collecting the copolyester of interest from the cultured cells or the culture. The culturing of a transformant for use in the production method of the present invention may generally be carried out by a method used for culturing the host cell.

As a culture medium when using a recombinant C. necator strain as the host, there can be mentioned a medium in which a microbially assimilable vegetable oil has been added and one of the nitrogen source, the inorganic salts, and the other organic nutrients has been limited. Typically, culturing is aerobically carried out for 1-10 days at a culture medium temperature of 25°C-37°C to allow the intracellular production and accumulation of the copolyester, and collecting and purifying it to prepare the desired copolyester.
C. necator, a parent strain of the transformant to be used in the production method of the present invention is known as a bacterium capable of growing with a vegetable oil as a carbon source. As a vegetable oil that can be used, a commercially available vegetable oil may generally be used, and its source may not be specifically limited. Preferably, there can be mentioned a natural oil such as a soybean oil, a corn oil, a safflower oil, a sunflower oil, an olive oil, a coconut oil, a palm oil, a rape oil, a fish oil, a whale oil, lard or tallow. In accordance with the present invention, the transformant of the present invention can be cultured at the same culture condition as the parent C. necator strain, and the concentration of the vegetable oil in the culture medium may preferably be 0.1-5%, but it could be adjusted as appropriate by a person skilled in the art.

Also, as needed, a nitrogen source or an inorganic substance may be added to the medium. As the nitrogen source, there can be mentioned ammonia, an ammonium salt such as ammonium chloride, ammonium sulfate and ammonium phosphate, peptone, meat extract, yeast extract, corn steep liquor and the like. As an inorganic substance, there can be mentioned monopotassium phosphate, dipotassium phosphate, magnesium phosphate, magnesium sulfate, sodium chloride and the like.

As the culture, a shaking culture may generally be used, and may be carried out at an aerobic condition at 25°C-37°C for at least one day after inducing gene expression. As the antibiotics, kanamycin, ampicillin etc. may be added to the culture medium. As an inducing agent of gene expression, as needed, arabinose, indoleacrylic acid (IAA), isopropyl-β-D-thiogalactopyraniside (IPTG) etc. may be used. A person skilled in the art could select the culture condition feasible for the expression of the desired gene, the condition for inducing gene expression, etc.

### (5) Purification and structural analysis of copolyester

In accordance with the present invention, a copolyester can be purified as follows: a transformant may be collected from the culture by centrifugation, washed with distilled water, and then dried and lyophilized. Subsequently, the dry transformant may be suspended in chloroform, stirred for a given period of time at room temperature, and then the copolyester may be extracted. During the extraction step, heating may be effected if needed. The residue may be removed by filtration, methanol may be added to the supernatant to precipitate the copolyester, and the precipitate may be filtered or centrifuged to remove the supernatant to obtain a dried and purified copolyester.

Means for confirming whether or not the copolyester obtained is the desired one may include, but not limited to, NMR (nuclear magnetic resonance) and gas chromatography. According to the present invention, the ratio of the monomer units of a copolyester, i.e. the ratio of 3-hydroxybutyric acid (3HB) and 3-hydroxyhexanoic acid (3HHx), can be adjusted as appropriate by varying the condition for gene expression, the culture condition etc. The fraction of 3HHx in the copolyester obtained by the production method of the present invention may preferably be 5-20 mol%, more preferably 5-15 mol%, and even more preferably 5-10 mol%. As used herein the term "mol%" refers to the mole of a certain component divided by the sum of moles of all components in a multi-component system. For example, when the general formula of a copolymer obtained by the production method of the present invention is expressed using "a" and "b" as the number of monomer units of 3HB and 3HHx, respectively, the fraction of 3HHx can be determined from the equation: a×100/(a+b).

### [Compound 1]

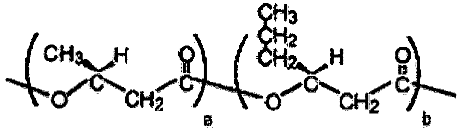

As shown in Example 18 below, copolyesters having different 3HHx fractions were obtained by the production method of the present invention (Tables 1 and 2, below). The copolyester produced by the production method of the present invention can be intracellularly accumulated at a rate of 50% by weight or more, preferably 55% by weight or more, per dry cell weight.

### Examples

The present invention will now be explained more specifically with reference to specific examples. But the present invention is not limited to the following examples below.

### Example 1

### C. necator strains used

In the Examples below, as a host for gene recombination, C. necator strain H16 (wild type, DSM428), strain H16Δ in which a PHA synthase gene phaC_{Cn} is deleted on the chromosome of the strain H16 (Mifune, J. et al., Can. J. Chem. (200), 86:621), and strain H16C_{Ac} strain (Mifune, J. et al., supra) in which the PHA synthase gene phaC_{Cn} on the chromosome of the H16 strain is replaced with the PHA synthase gene phaC_{Ac} derived from a soil bacterium A. caviae.

### Example 2

### Cloning of a doubly amino acid-mutated PHA synthase gene

Mutant enzymes in which two amino acid mutations were introduced in the PHA synthase derived from A. caviae are reported (Tsuge, T. et al., FEMS Microbiol. Lett (2007), 277:217-222; Kichise et al., Appl. Environ. Microbiol. (2002), 68:2411-2419; Kokai (Japanese Unexamined Patent Publication) No. 2008-29218). As used herein, the mutant PHA synthase gene may be termed as "phaC_{NSDG}." Using pBBREE32-NSDG (Tsuge, T. et al., supra) in which phaC_{NSDG} was cloned as the template, phaC_{NSDG} was amplified by a PCR method with the oligonucleotides of the following sequence 1 and sequence 2 as the primers. Using the KOD Plus for PCR (manufactured by Toyobo), a cycle comprising 20 seconds at 98°C, 15 seconds at 61°C and 2 minutes at 68°C was repeated for 30 cycles.
Sequence 1: GGTTCGAATAGTGACGGCAGAGAGACAATCAAATCATGAGCCAACCATCTTATGGC (SEQ ID NO: 5) (underlined is the Csp45I restriction enzyme site)
Sequence 2: AACCTGCAGGCCTGCCGGCGCCGTGCATATGCAAGCGTCATGCGGCGTCCTCCTCTG (SEQ ID NO: 6) (underlined is the SbfI restriction enzyme site)

The amplified phaC_{NSDG} fragment was added to the 3'-end using the TA Cloning kit (manufactured by Toyobo), and ligated to the multi-cloning site of a vector plasmid pTA2 (manufactured by Toyobo) using the Ligation High (manufactured by Toyobo) to construct pTA2_{NSDG}.

### Example 3

### Construction of vector for introducing the doubly amino acid-mutated PHA synthase

pTA2_{NSDG} was cleaved with Csp45I and SbfI to isolate a gene fragment containing pha2_{NSDG}. Subsequently, pTA2C'AB (Mifune, J. et al., supra) containing a recombinant pha operon (promoter region-phaC_{Ac}-phaA_{Cn}-phaB1_{Cn}) was cleaved with Csp45I and SbfI, the 5'-end was dephosphorylated with alkaline phosphatase (manufactured by Toyobo), and then the previously obtained phaC_{NSDS} fragment was inserted to obtain pTA2_{NSDG}-AB- Herein, phaB1_{Cn} is an acetoacetyl-CoA reductase gene on the chromosome of C. necator strain. By cleaving pTA2_{NSDG}-AB with BamHI, a 5.0 kb gene segment harboring the promoter region-phaC_{NSDG}-phaA_{Cn}-phaB1_{Cn} was isolated. Also, pK18mobsacB (Schafer et al., Gene (1994), 147:198) was cleaved with BamHI, and the 5'-end was dephosphorylated with alkaline phosphatase. Using the Ligation High, two fragments were ligated to construct pK18msNSDG-AB.

### Example 4

### Construction of NSDG strain

Using the recombinant plasmid pk18msNSDG-AB obtained, C. necator strain H16ΔC was transformed by transconjugation. First, by the calcium chloride method, pk18msNSDG-AB was introduced into E. coli strain S17-1. Then, this recombinant E. coli was cultured overnight at 37°C in 3.0 ml of LB medium (1% tryptone, 1% sodium chloride, 0.5% yeast extract, pH 7.2). In parallel to this, C. necator strain H16ΔC was cultured overnight at 30°C in 3.0 ml of NR medium (1% fish meat extract, 1% polypeptone, 0.2% yeast extract). Then, to 0.2 ml of the E. coli culture, 0.1 ml of the culture of C. necator strain H16ΔC was mixed, and cultured for 6 hours at 30°C. The cell mixture was plated on the Simmons Citrate agar medium (manufactured by Difco) with 0.2 mg/ml kanamycin, and cultured at 30°C for 3 days. Cells in which the plasmid of recombinant E. coli was transferred to C. necator and incorporated on the chromosome by homologous recombination exhibited resistance to kanamycin. On the other hand, since the recombinant E. coli cannot grow in the Simmon's Citrate agar medium, the colonies that grew in the above medium are C. necator transformants (pop-in strain) in which pK18msNSDG-AB was incorporated from the recombinant E. coli into the chromosome. Furthermore, after culturing the pop-in strain in NR medium overnight at 30°C, it was plated on NR medium with 10% sucrose and cultured at 30°C for 3 days. Levan sucrase encoded by sacB on pK18mobsacB intracellularly accumulates toxic polysaccharides using sucrose as the substrate. Thus, in NR medium with 10% sucrose, the strain (pop-out strain) in which the plasmid segment was eliminated can only grow. Clones in which phaC_{NSDG} was inserted from these colonies onto the chromosome were selected by a PCR method and were designated as C. necator strain NSDG. This NSDG strain was used as the host as appropriate in gene recombination below.

### Example 5

### Construction of vector (for the H16C_{Ac} strain) for destructing phaA_{Cn}-phaB1_{Cn}

Using the genomic DNA of C. necator strain H16 as the template, 1041 bp gene segment comprising a transcription regulator gene (phaR) downstream to phaB1_{Cn} was amplified by a PCR method with the following oligonucleotides of sequence 3 and sequence 4 as the primers.
Sequence 3: TCGACCGGCGCCGACTTCTC (SEQ ID NO: 7)
Sequence 4: GCATGCCAGTGTCTTACTTCT (SEQ ID NO: 8) (underlined is the SphI restriction enzyme site)

Using the KOD Plus for PCR (manufactured by Toyobo), a cycle comprising 20 seconds at 98°C, 15 seconds at 65°C and 1 minute 15 seconds at 68°C was repeated for 30 cycles. The amplified fragment was purified by the DNA Purification kit (manufactured by Promega). Then, the 5'-end was phosphorylated with T4 kinase (manufactured by Toyobo) and ligated to pUC118 (manufactured by Takara-Bio) treated with HincII and alkaline phosphatase using the Ligation High. The constructed plasmid was cleaved with NdeI and SphI to isolate a gene segment comprising the downstream of phaB1_{Cn}. pK18C'AB (Mifune, J. et al., supra) in which a recombinant pha operon (promoter region-phaC_{AC}-phaA_{Cn}-phaB1_{Cn}) had been inserted to pK18mobsacB was cleaved with NdeI and SphI to remove the phaA_{Cn}-phaB1_{Cn} segment. By ligating the above gene segment containing the downstream of phaB1_{Cn}, pK18msC'R harboring the promoter region-phaC_{Ac}-phaR was obtained. Furthermore, pK18msC'R was cleaved with EcoRI and Csp45I, and after the ends were blunt-ended using the Blunting High (manufactured by Toyobo), it subjected to self ligation using the Ligation High to remove the promoter region and part of phaC_{Ac} to obtain pK18msC'RΔP in which the lengths of two homologous regions flanking phaA_{Cn}-phaB1_{Cn} were controlled,

### Example 6

### Construction of vector (for the NSDG strain) for destructing phaA_{Cn}-phaB1_{Cn}

By cleaving pK18NSDG-AB with NdeI and SphI and ligating thereto the above gene segment comprising the downstream of phaB1_{Cn}, pK18msNSDG-R harboring the promoter region-phaC_{NSDG}-phaR was obtained. Furthermore, pK18msNSDG-R was cleaved with EcoRI and Csp45I, and after the ends were blunt-ended using Blunging High, it was subjected to self ligation using the Ligation High to remove the promoter region and part of phaCA_{c} to obtain pK18msNSDGRΔP in which the lengths of two homologous regions flanking phaA_{Cn}-phaB1_{Cn} were controlled.

### Example 7

### Construction of vector for destructing phaB1

Using pTA2NSDG-AB as the template, a gene segment comprising phaA_{Cn} was amplified by a PCR method with the oligonucleotides of the following sequence 5 and sequence 6 as the primers. Using the KOD Plus for PCR, a cycle comprising 20 seconds at 98°C, 15 seconds at 65°C and 1 minute 20 seconds at 68°C was repeated for 30 cycles.
Sequence 5: CGCCGCATGACGCTTGCATA (SEQ ID NO: 9)
Sequence 6: CCATATGCGGCCCCGGAAAACCCC (SEQ ID NO: 10) (underlined is the NdeI restriction enzyme site)

Then, the 5'-end was phosphorylated with T4 kinase and ligated using the Ligation High to pUC118 treated with HincII and alkaline phosphatase. The plasmid obtained was cleaved with NdeI to isolate a gene segment comprising phaA_{Cn}. Also, pK18msC'R was cleaved with NdeI, and the 5'-end was dephosphorylated by alkaline phosphatase treatment. By ligating the fragment obtained using the Ligation High, pK18msC'AR harboring the promoter region-phaC_{NSDG}-phaA_{Cn}-phaR was obtained. Furthermore, pK18msC'AR was cleaved with EcoRI and XhoI to remove the promoter region and the phaC_{NSDG} region. Then, using the Blunting High, the ends were blunt-ended and then subjected to self ligation using the Ligation High to obtain pK18msAR.

### Example 8

### Construction of vector (for the H16C_{Ac} strain) for destructing phaA_{Cn}

Using pTA2NSDG-AB as the template, a gene segment comprising phaB1_{Cn} was amplified by a PCR method with the oligonucleotides of the following sequence 7 and sequence 8 as the primers. Using the KOD Plus for PCR, a cycle comprising 20 seconds at 98°C, 15 seconds at 65°C and 1 minute at 68°C was repeated for 30 cycles.
Sequence 7: CGCATATGGTTGGCGCGGA (SEQ ID NO: 11) (underlined is the NdeI restriction enzyme site)
Sequence 8: GCCCGGATCCTATGCCCAACAAGGCACTAAG (SEQ ID NO: 12) (underlined is the BamHI restriction enzyme site)

Then, the 5'-end was phosphorylated with T4 kinase and ligated using the Ligation High to pUC118 (manufactured by Toyobo) treated with HincII and alkaline phosphatase. Furthermore, the plasmid constructed was cleaved with NdeI to isolate a gene segment comprising phaB1_{Cn}. Also, pK18msC'R was cleaved with NdeI, and by alkaline phosphatase treatment, the 5'-end was dephosphorylated. By ligating the fragment obtained, pK18msC'BR harboring the promoter region-phaC_{NSDG}-phaB1_{Cn}-phaR was obtained.

### Example 9

### Construction of vector (for the NSDG strain) for destructing phaA_{Cn}

By cleaving pK18msNSDG-R (Example 6) with NdeI and ligating thereto the phaB1 fragment obtained as above (Example 8), pK18msNSDG-BR harboring the promoter region-phaC_{NSDG}-phaB1_{Cn}-phaR was obtained.

### Example 10

### Construction of vector for introducing R-hydratase gene (phaJ_{Ac}) derived from A. caviae

Using the genomic DNA of C. necator strain H16 as the template, a 1 kbp gene segment upstream to phaA_{Cn} (containing part of phaC_{NSDG}) was amplified by a PCR method with the oligonucleotides of sequence 9 and sequence 10 as the primers. Using the KOD Plus for PCR, a cycle comprising 20 seconds at 98°C, 15 seconds at 65°C and 1 minute 10 seconds at 68°C was repeated for 30 cycles. Also, with the oligonucleotides of sequence 11 and sequence 12 as the primers, an about 1 kbp gene segment downstream to phaA_{Cn} containing phaB1_{Cn} was amplified under the same PCR condition as above. The two gene fragments obtained were purified by the DNA Purification kit, and fusion PCR was carried out with a solution in which 100 ng each was mixed as the template. The ends of the two fragments are complementary by the 26 bp complementary sequence designed in sequence 10 and sequence 11. After PCR reaction (a cycle comprising 20 seconds at 98°C, 15 seconds at 65°C and 2 minutes 10 seconds at 68°C was repeated for 10 cycles) was carried out without primer addition, the oligonucleotides represented by sequence 9 and sequence 12 were added as the primers, and after further adding 0.5 µl of the KOD Plus, a cycle comprising 20 seconds at 98°C, 15 seconds at 65°C and 2 minutes 10 seconds at 68°C was repeated for 30 cycles.
Sequence 9: TGCGAATTCAGAACTCCCTGGTCGCCT (SEQ ID NO: 13) (underlined is the EcoRI restriction enzyme site)
Sequence 10: CGTCGGATCCTCTAGATTCGAAGCGTCATGCGGCGTCCTCCTCTG (SEQ ID NO: 14) (underlined is the BamHI-XbaI-Csp45I, nucleotide 1-26 is a complementary sequence to sequence 11)
Sequence 11: ACGCTTCGAATCTAGAGGATCCGACGATAACGAAGCCAATCAAGG (SEQ ID NO: 15) (Underlined is Csp45I-XbaI-BamHI, nucleotide 1-26 is a complementary sequence to sequence 10)
Sequence 12: TGCGCAAGCTTTATGCCCAACAAGGCACTAAGAAAAG (SEQ ID NO: 16) (underlined is the HindIII restriction enzyme site)

A 2 kbp gene fragment in which 1 kbp fragments upstream and downstream to phaA_{Cn} were ligated was excised from the agarose gel, purified by the DNA Purification kit, and further cleaved with EcoRI and HindIII. Also, pK18mobsacB was cleaved with EcoRI and HindIII, and treated with alkaline phosphatase to carry out 5'-dephosphorylation. By ligating these fragments with the Ligation High, pK18ms NSDG-B harboring a gene segment in which part of phaC_{NSDG} and were ligated was obtained.

Subsequently, using pEE32 (Fukui, T. et al., J. Bacteriol. (1997), 179:4821) as the template, a R form-specific enoyl-CoA hydratase gene (phaJ_{Ac}) derived from A. caviae was amplified by a PCR method with the following sequence 13 and sequence 14 as the primer.
Sequence 13: CATCTCCTGCAGGTTCGAAGAGGAGGACGCCGCATGAGCGCACAATCCCTGGAAGTA GGCCAGA (SEQ ID NO: 17) (underlined is the SbfI, and nucleotide 14-19 is the Csp45I restriction enzyme site)
Sequence 14: CGCCACCTGCAGGCTCTAGATTAAGGCAGCTTGACCACGGCTT (SEQ ID NO: 18) (underlined is the SbfI, and nucleotide 15-20 is the XbaI restriction enzyme site)

The gene fragment obtained was cleaved with SbfI, and purified by isopropanol precipitation. Also, pTA2NSDG-AB (described in Example 3) was cleaved with SbfI, the 5'-end was dephosphorylated with alkaline phosphatase treatment, and then ligated to the phaJ fragment by the Ligation High to obtain pTA2-NSDG-JAB. By cleaving pTA2-NSDG-JAB with BamHI, a 5.4 kb fragment containing part of phaC_{NSDG}-phaJ_{Ac}-phaA_{Cn}-phaB1_{Cn} was ligated to the BamHI site of pK18mobsacB to construct pK18msNSDG-JAB.

### Example 11

### Construction of vector for deleting phaA_{Cn} and introducing phaJ_{AC}

The phaJ_{AC} fragment obtained in Example 10 was cleaved with Csp45I and XbaI, and purified by isopropanol precipitation. By ligating this fragment using the Ligation High to pK18msNSDG-B cleaved with Csp45I and XbaI, pK18msNSDG-JB harboring a gene fragment in which part of phaC_{NSDG} and phaJ_{AC} and phaB1_{Cn} are ligated was obtained.

### Example 12

### Construction of vector for deleting phaA_{Cn} and introducing bktB_{Cn}

Using genomic DNA of C. necator strain H16 as the template, bktB_{Cn} gene segment was amplified by the PCR method with the following oligonucleotides of sequence 15 and sequence 16 as the primers.
Sequence 15: TACATTCTAGAAAGGAGGCAAAGTCATGACGCGTGAAGTGGTAGTGGTA (SEQ ID NO: 19) (underlined is the XbaI restriction enzyme site)
Sequence 16: GCTCAGGATCCACCCCTTCCTCAGATACGCTCGAAGATGGCGG (SEQ ID NO: 20) (underlined is the BamHI restriction enzyme site)

Using the KOD Plus (manufactured by TOYOBO) for PCR, a cycle comprising 20 seconds at 98°C, 15 seconds at 65°C and 1 minute 15 seconds at 68°C was repeated for 30 cycles. The amplified fragment was purified by the DNA Purification kit. Then it was cleaved with XbaI and BamHI and purified by isopropanol precipitation. Also, pK18NSDG-B (Example 10) was cleaved with XbaI and BamHI, and then subjected to alkaline phosphatase treatment. The two fragments were ligated using the Ligation High to obtain pK18msNSDG-bB harboring part of phaC_{NSDG}-bktB_{Cn}-phaB1_{Cn}.

### Example 13

### Construction of vector for deleting phaA_{Cn} and introducing phaJ_{AC} ·bktB_{Cn}

pK18msNSDG-JB (Example 11) was cleaved with XbaI and HindIII to isolate a gene fragment containing phaJ_{Ac}-phaB1_{Cn}. Also, pK18msNSDG-bB (Example 12) was cleaved with XbaI and HindIII, and a gene fragment containing a vector segment was excised from agarose gel, and purified. The two fragments were ligated using the Ligation High to obtain pK18msNSDG-JbB harboring part of phaC_{NSDG}-phaJ_{Ac}-bktB_{Cn}-phaB1_{Cn}.

### Example 14

### Construction of a homologous recombinant strain

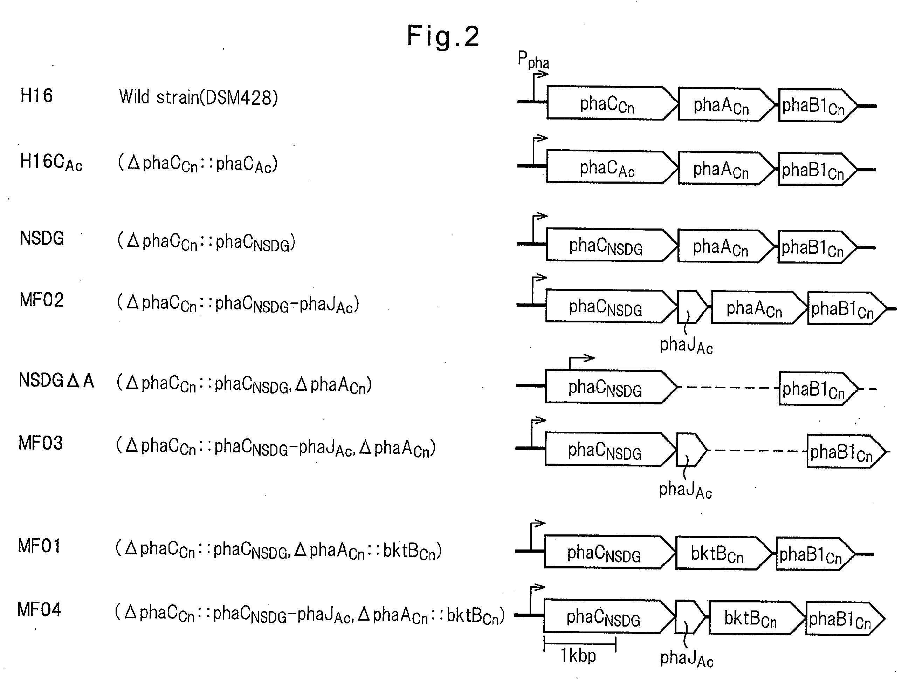

Using the plasmid for homologous recombination constructed in Example 13 in a method similar to Example 4, a C. necator strain was transformed by transconjugation, and then clones in which the desired recombination occurred were selected by a PCR method. Thus, using pK18msC'BR (Example 8), pK18AR (Example 7) and pK18msC'RΔP (Example 5) with the H16C_{Ac} strain as the host, the H16C_{Ac}ΔA strain, the H16C_{Ac}AB strain, and the H16C_{Ac}ΔAB strain in which phaA_{Cn}, and phaA_{Cn}-phaB1_{Cn}, respectively, were destructed were constructed. Also, using pK18msNSDG-BR (Example 9), pK18msAR (Example 7) and pK18msNSDG-RΔP (Example 6) with the NSDG strain as the host, the NSDGΔA strain, the NSDGΔB strain, and the NSDGΔAB strain in which phaA_{Cn}, and phaA_{Cn}-phaB1_{Cn}, respectively, were destructed were constructed. Also, the MF01 strain in which phaA_{Cn} was replaced with bktB_{Cn} using pK18msNSDG-bB (Example 12), the MF02 strain (Example 10) in which phaJ_{AC} was inserted in between phaC_{NSDG} and phaA_{Cn} using pK18msNSDG-JAB, the MF03 strain (Example 11) in which phaA_{Cn} was replaced with phaJ_{Ac} using pK18msNSDG-JB, and the MF04 strain in which phaA_{Cn} was replaced with phaJ_{Ac}-bktB_{Cn} using pK18msNSDG-JbB (Example 13) were constructed (see Fig. 2).

### Example 15

### Construction of plasmid for gene expression

Using the genomic DNA of C. necator strain H16 as the template, a 0.5 kbp gene segment (the PhaP promoter region) upstream to the PHA granule binding protein gene (phaP_{Cn}) was amplified by a PCR method with the oligonucleotides of the following sequence 17 and sequence 18 as the primers.
Sequence 17: AAAGATCTAATATTGTTCTGTTCACGTCTTTGTTAGTTCC (SEQ ID NO: 21) (underlined is the BglII-SspI restriction enzyme site)
Sequence 18: GAGGATCCCCGGGTACCTATGAATTCATATGTATATACCTCCCAGGCGTGTGGGTGG GTCAAG (SEQ ID NO: 22) (underlined is the BamHI restriction enzyme site)

Using the KOD Plus for PCR, a cycle comprising 20 seconds at 98°C, 15 seconds at 65°C and 30 seconds at 68°C was repeated for 30 cycles. The amplified fragment was purified by the DNA Purification kit. Then the 5'-end was phosphorylated with T4 kinase, and ligated using the Ligation High to HincII- and alkaline phosphatase-treated pUC118. The constructed plasmid was further cleaved with BglII and BamHI to isolate a DNA fragment of the PhaP promoter region. Also, plasmid pBAD24 (GenBank Accession No. X81837) was cleaved with a restriction enzyme BamHI to remove the BAD promoter region, and an alkaline phosphatase-treated fragment and the above PhaP promoter region were ligated using the Ligation High. By cleaving the plasmid obtained with a restriction enzyme SspI, a DNA fragment containing a phaP promoter region, a multicloning site and a rrnB terminator region was isolated. Also, a broad-host-range vector pBBR1-MCS2 (GenBank Accession No. U23751) known to be capable of autonomously growing in various gram negative bacteria was cleaved with a restriction enzyme SspI to isolate a DNA fragment containing the origin of replication. After subjecting to dephosphorylation treatment, it was ligated to a DNA fragment containing the above phaP promoter region using the Ligation High to construct an expression plasmid pBBRPP.

### Example 16

### Cloning of R form-specific enoyl CoA hydratase gene (phaJ1, phaJ2, phaJ3) derived from C. necator

By homology search with a known R form-specific enoyl CoA hydratase, phaJ1 (SEQ ID NO: 2), phaJ2 (SEQ ID NO: 3), and phaJ3 were identified as a presumptive R form-specific enoyl CoA hydratase gene. Then, using the genomic DNA of C. necator as the template, phaJ1 was amplified by a PCR method with the oligonucleotides of the following sequence 19 and sequence 20 as the primers. Similarly, phaJ2 was amplified by a PCR method with the oligonucleotides of sequence 21 and sequence 22 as the primers. Furthermore, phaJ3 was amplified by a PCR method with the oligonucleotides of sequence 23 and sequence 24 as the primers. Using the KOD Plus (manufactured by TOYOBO) for PCR, a cycle comprising 20 seconds at 98°C, 15 seconds at 61°C and 2 minutes at 68°C was repeated for 30 cycles.
Sequence 19: TCCATATGCGTACCATCGCATCGCTG (SEQ ID NO: 23) (underlined is the NdeI restriction enzyme site)
Sequence 20: CGCTCGAGTCACCCGTAGCGGCGCGTGA (SEQ ID NO: 24) (underlined is the XhoI restriction enzyme site)
Sequence 21: CACATATGAAGACCTACGAGAACATC (SEQ ID NO: 25) (underlined is the NdeI restriction enzyme site)
Sequence 22: CTCGAGTCAGGGAAAGCGCCGCAGGA (SEQ ID NO: 26) (underlined is the XhoI restriction enzyme site)
Sequence 23: CACATATGCCAAGAATCTTCCGTTCT (SEQ ID NO: 27) (underlined is the NdeI restriction enzyme site)
Sequence 24: ATCTCGAGTCAGAAGTAATAGCGGCTG (SEQ ID NO: 28) (underlined is the XhoI restriction enzyme site)

The amplified fragment was cleaved with NdeI and XhoI, and ligated using the Ligation High to an expression vector pBBRPP (Example 15) cleaved with NdeI and SalI to obtain pBBR-phaJ1, pBBR-phaJ2 and pBBR-phaJ3, expression vectors of phaJ1, phaJ2 and phaJ3, respectively, derived from C. necator.

### Example 17

### Construction of C. necator-derived phaJ-expressing strain

The expression vectors containing the above R-hydratase gene was transformed into C. necator strain H16C_{Ac} or strain NSDGΔ (Example 14) by transconjugation. First, pBBR-phaJ1, pBBR-phaJ2 or pBBR-phaJ3 was introduced into E. coli strain S17-1 by the calcium chloride method. Then, this recombinant E. coli was cultured overnight at 37°C in 3.0 ml of LB medium (1% tryptone, 1% sodium chloride, 0.5% yeast extract, pH 7.2). In parallel to this, a C. necator strain was cultured overnight at 30°C in 3.0 ml of NR medium (1% fish meat extract, 1% polypeptone, 0.2% yeast extract). Then, to 0.2 ml of the E. coli culture, 0.1 ml of the culture of the C. necator strain was mixed, and cultured for 6 hours at 30°C. The cell mixture was plated on the Simmon's Citrate agar medium (manufactured by Difco) with 0.2 mg/ml kanamycin, and cultured for 3 days at 30°C. The plasmid of recombinant E. coli was transferred into C. necator, and the cells carrying the plasmid exhibited resistance to kanamycin. On the other hand, since the recombinant E. coli cannot grow in the Simmon's Citrate agar medium, the colonies that grew in the above medium are C. necator transformants. By confirming the retention of the vector by PCR, each of the H16C_{Ac}/pBBR-phaJ1 strain, the H16C_{Ac}/pBBR-phaJ2 strain, the H16C_{Ac}/pBBR-phaJ3 strain, the NSDGΔA/pBBR-phaJ1 strain, the NSDGΔA/pBBR-phaJ2 strain, and the NSDGΔA/pBBR-phaJ3 strain was isolated.

### Example 18

### Synthesis of copolyester with recombinant C. necator strain

A recombinant C. necator strain precultured in NR medium (described above) was inoculated in 100 ml of MB medium (0.9% disodium hydrogen phosphate·12 H2O, 0.15% potassium dihydrogen phosphate, 0.05% ammonium chloride, 1% trace metal solution), and cultured under shaking in a shaking flask at 30°C for 72 hours. As a carbon source, 1% soybean oil was used. For a recombinant strain harboring an expression vector, pBBR-phaJ1, pBBR-phaJ2, or pBBR-phaJ3, 0.1 mg/ml kanamycin was added to the culture medium. After completion of culturing, the cells were collected, and, after washing with 70% ethanol to remove oils attached, washed with distilled water. The cells obtained were lyophilized and the dry cell weight was measured.

To 10-30 mg of the dry cells, 2 ml of sulfuric acid-methanol mixture (15:85) and 2 ml of chloroform were added, and sealed tightly. BY heating at 100°C for 140 minutes, the methyl ester of the intracellular polyester degradation in the cell was obtained. One ml of distilled water was added thereto, and shaken vigorously. After allowing to stand so as to separate into two layers, the bottom organic layer was removed. 0.5 ml of the organic layer was analyzed by capillary gas chromatography. The gas chromatography used is GC-17A manufactured by Shimadzu, and the capillary column used is InertCap-1 manufactured by GL Science (column length 25 m, the inner diameter of the column 0.25 mm, the thickness of the liquid film 0.4 µm). The temperature condition comprised a temperature rise at 8°C/min from an initial temperature of 100°C. The results obtained are shown in the following Tables 1 and 2.

### [Table 1]

**Table 1. PHA production by a recombinant C. necator having introduced therein A. caviae-derived R-hydratase gene with 1% soybean oil as a carbon source**

| Strain | | Dry cell weight (g/l) | Amount of PHA produced (g/l) | PHA content (wt%) | 3HHx fraction (mol%) |
|---|---|---|---|---|---|
| NSDG | | 6.61 | 5.68 | 85.9 | 1.90 |
| MFO2 | phaJ_{Ac}-introduced | 6.82 | 5.51 | 80.8 | 5.90 |
| NSDGΔA | | 4.51 | 3.38 | 75.0 | 3.20 |
| MF03 | phaJ_{Ac}-introduced | 4.80 | 3.93 | 81.8 | 9.20 |
| MF01 | | 5.07 | 4.14 | 81.6 | 2.60 |
| MF04 | phaJ_{Ac}-introduced | 6.00 | 4.88 | 81.3 | 7.00 |

### [Table 2]

**Table 2. PHA production by a recombinant C. necator having introduced therein C. necator-derived R-hydratase gene with 1% soybean oil as a carbon source**

| Host | Plasmid introduced | | Dry cell weigh t (g/l) | Amount of PHA produced (g/1) | PHA content (wt%) | 3HHx fractio n (mol%) |
|---|---|---|---|---|---|---|
| H16C_{Ac} | pBBRPP | Control | 2.74 | 1.99 | 72.8 | 0.67 |
| H16C_{Ac} | pBBRPP-phaJ1 | phaJ1-introduced | 2.34 | 1.71 | 72.9 | 1.31 |
| H16C_{AC} | pBBRPP-phaJ2 | phaJ1-introduced | 2.04 | 1.39 | 68.3 | 1.17 |
| H16C_{AC} | pBBRPP-phaJ3 | phaJ1-introduced | 2.37 | 1.64 | 69.0 | 0.86 |
| NSDGΔA | pBBRPP | Control | 2.71 | 1.98 | 73.1 | 2.56 |
| NSDGΔA | pBBRPP-phaJ1 | phaJ1-introduced | 2.29 | 1.33 | 58.2 | 8.90 |
| NSDGΔA | pBBRPP-phaJ2 | phaJ1-introduced | 1.87 | 1.25 | 67.2 | 7.69 |
| NSDGΔA | pBBRPP-phaJ3 | phaJ1-introduced | 1.87 | 1.24 | 66.2 | 2.69 |

All the strains in which the phaJ_{Ac} gene was introduced into the chromosome had significantly enhanced 3HHx fractions in P(3HB-co-3HHx) while maintaining high dry cell weight and high PHA accumulation rate. Specifically the 3HHx fraction in P(3HB-co-3HHx) synthesized in the MF03 strain reached as high as 9.2 mol%, which may be expected to have sufficient flexibility for putting into practical use. When C. necator-derived phaJ was introduced using a self replicating vector, the dry cell weight and the PHA accumulation rate tended to slightly decrease, but in all the host strains, the introduction of the phaJ1 gene or the phaJ2 gene led to the increased 3HHx fraction in P(3HB-co-3HHx). Specifically, in strains in which the phaJ1 gene or the phaJ2 gene was introduced with the NSDGΔA strain as the host, the 3HHx fraction reached 7.7-8.9 mol%, which may be expected to have sufficient flexibility for putting into practical use. No clear effect was observed in the phaJ3 gene-introduced strains.

### Example 19

### Construction of vector for introducing phaJ1, phaJ2

Using pBBR-phaJ1 (Example 16) as the template, the phaJ1 fragment was amplified by a PCR method with the oligonucleotides of sequence 25 and sequence 26 as the primers. Also, using pBBR-phaJ2 (Example 16) as the template, the phaJ2 fragment was amplified by a PCR method with the oligonucleotides of sequence 27 and sequence 28 as the primers.
Sequence 25: TTGACACTAGTTCTAGAAGGAGGAGTATATACATATGCGTACCATCGCATCGC (SEQ ID NO: 29) (underlined is the XbaI restriction enzyme site)
Sequence 26: GCCAAGCGGCCGCTTCGAAACTAGTTGCAGCTCGACTCACCCGTAG (SEQ ID NO: 30) (underlined is the SpeI restriction enzyme site)
Sequence 27: GACCCACTAGTTCTAGAAGGAGGAATATACATATGAAGACCTACGAGAACAT (SEQ ID NO: 31) (underlined is the XbaI restriction enzyme site)
Sequence 28: CAAGCGCGGCCGCTTCGAAACTAGTTCAGGGAAAGCGCCGCAGGAT (SEQ ID NO: 32) (underlined is the SpeI restriction enzyme site)

Using the KOD Plus (TOYOBO) for PCR, a cycle comprising 20 seconds at 98°C, 20 seconds at 60°C and 50 seconds at 68°C was repeated for 30 cycles. The amplified fragment was purified by the DNA Purification kit. Then the 5'-end was phosphorylated with T4 kinase, and ligated using the Ligation High to HincII- and alkaline phosphatase-treated pUC118. The constructed plasmid was further cleaved with XbaI and SpeI to isolate gene segments comprising phaJ1 and phaJ2 fragments. Also pK18msNSDG-B (Example 10), after cleaving with XbaI, was subjected to dephosphorylation treatment, to which the above phaJ1 fragment and phaJ2 fragment were ligated using the Ligation High. Those in which the directions of phaC_{NSDG} and phaJ1 and phaJ2 are the same were selected, and designated as pK18msNSDG-J1 and pK18msNSDG-J2.

Similarly pK18msNSDG-JB (Example 11), after cleaving with XbaI, was subjected to dephosphorylation treatment, to which the above phaJ1 fragment and phaJ2 fragment were ligated using the Ligation High. Those in which the directions of phaC_{NSDG} and phaJ1 and phaJ2 are the same were selected, and designated as pK18msNSDG-JAcJ1B and pK18msNSDG-JAcJ2B.

### Example 20

### Construction of a homologous recombinant strain

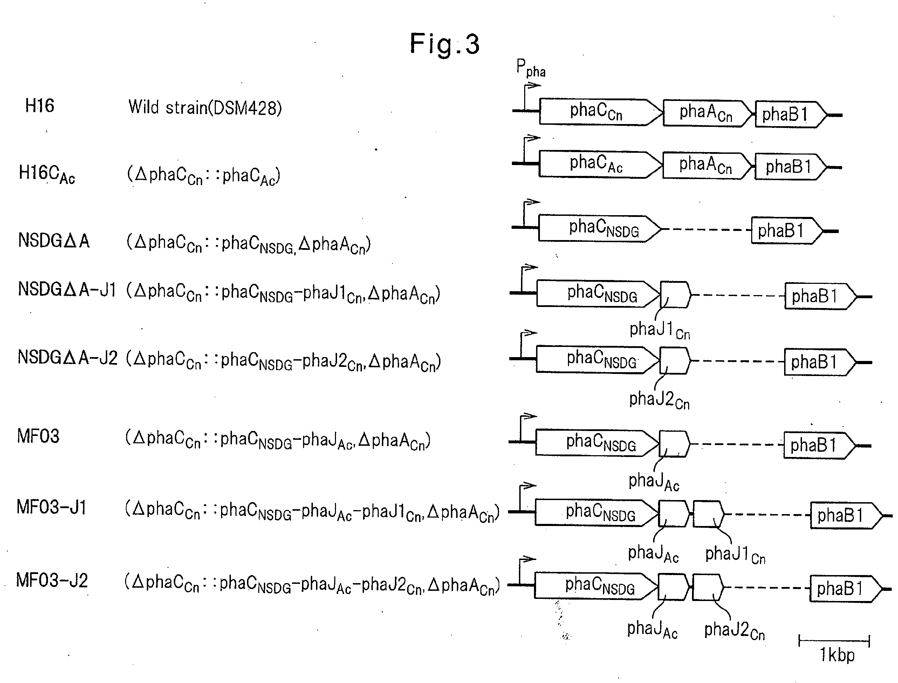

Using the plasmids for homologous recombination constructed in the above Examples in a method similar to Example 4, the C. necator strain was transformed by transconjugation, and then clones in which the desired recombination occurred were selected by a PCR method. Thus, using the NSDG strain as the host, the NSDGΔA-J1 strain in which phaA was replaced with phaJ1 using pK18msNSDG-J1B (Example 19), the MF03-J1 strain in which phaA was replaced with phaJAc-phaJ1 using pK18msNSDG-JAcJ1B (Example 19), the NSDGΔA-J2 strain in which phaA was replaced with phaJ2 using pK18msNSDG-J2B (Example 19), and the MF03-J2 strain in which phaA was replaced with phaJAc-phaJ2 using pK18msNSDG-JAcJ2B (Example 19) were constructed. The structure of gene arrangement of each strain is shown in Fig. 3.

### Example 21

### Synthesis of copolyester with recombinant C. necator

The synthesis of copolyester with a recombinant C. necator constructed in Example 20 was carried out in a manner similar to Example 18. The result is shown in Table 3. In the NSDGΔA-J1strain and the NSDGΔA-J2 strain, compared to the control NSDGΔA strain, the 3HHx fraction increased from 3.2 mol% to 7.3-8.0 mol% without a decrease in the amount of PHA produced. This 3HHx fraction was slightly lower than that of the MF03 strain (Example 14) in which phaJAc, the R-enoyl-CoA hydratase gene derived from A. caviae, was introduced by homologous recombination. On the other hand, in the MF03-J1 strain and MF03-J2 strain in which phaJ was arranged in tandem with phaJ1 and phaJ2, respectively, the 3HHx fraction was improved to 10.2-10.5 mol% while keeping the high amount of PHA produced.

### [Table 3]

**Table 3. PHA production by a recombinant C. necator having introduced therein C. necator-derived R-hydratase gene with 1% soybean oil as a carbon source**

| Strain | | Dry cell weigh t (g/l) | Amount of PHA produce d (g/l) | PHA content (wt%) | 3HHx fractio n (mol%) |
|---|---|---|---|---|---|
| NSDGΔA | phaA-deleted | 4.51 | 3.38 | 75.0 | 3.20 |
| MF03 | phaA-deleted,phaJ_{Ac}-introduced | 4.80 | 3.93 | 81.8 | 9.20 |
| NSDGΔA-J1 | phaA-deleted, phaJ1_{Cn}-introduced | 4.74 | 3.85 | 81.3 | 7.25 |
| NSDGΔA-J2 | phaA-deleted, phaJ2_{Cn}-introduced | 4.87 | 3.99 | 82.0 | 7.98 |
| MF03-J1 | phaA-deleted, phaJ_{Ac}·phaJ1_{Cn}-introduced | 4.73 | 3.89 | 82.4 | 10.5 |
| MF03-J2 | phaA-deleted, phaJ_{Ac}·phaJ2_{cn}-introduced | 4.75 | 3.85 | 81.1 | 10.1 |

### Example 22

### Functional analysis of R form-specific enoyl CoA hydratase derived from C. necator

Each fragment of phaJ1, phaJ2 and phaJ3 that were amplified and cleaved with NdeI and XhoI in Example 16 was inserted into an expression plasmid pCOLDII (manufactured by Takara-Bio) cleaved with each of NdeI and XhoI. The plasmids obtained were designated as pCOLD-J1, pCOLD-J2 and pCOLD-J3. These expression plasmids have been constructed so as to produce recombinant protein in which a tag sequence (hereinafter referred to as His tag) comprising 6 histidine residues has been added to the amino terminal end. Using pCOLD-J1, pCOLD-J2 and pCOLD-J3, E. coli strain BL21 (DE3) (manufactured by Novagen) was transformed. Each transformant obtained was cultured in 100 mL of LB medium with 100 mg ampicillin at 37°C for 4 hours, to which isopropyl-thiogalacto-pyranoside (IPTG) was added at a final concentration of 0.5 mM to induce expression, and further culcuted at 15°C for 24 hours. The cells collected by centrifugation were resuspended in 20 mM sodium phosphate buffer (pH 7.4) containing 30 mM imidazole and 500 mM NaCl, sonic-disrupted and centrifuged to obtain soluble protein fractions.

This soluble protein fraction was filtered with a filter having a pore size of 0.22 µm. Then, this was subjected to the HisTrap FF crude column (1 ml) (manufactured by GE Healthcare) equalized in advance with 20 mM sodium phosphate buffer (pH 7.4) containing 30 mM imidazole and 500 mM NaCl so as to allow the His tag-carrying protein to be adsorbed. Subsequently, by linearly increasing the imidazole concentration from 30 mM to 500 mM, the adsorbed protein was eluted and recovered to prepare purified enzyme.

Proteins expressed from the three genes, phaJ1, phaJ2 and phaJ3, derived from C. necator are hereinafter referred to as PhaJ1, PhaJ2 and PhaJ3, respectively. For each of purified recombinant protein products produced in E. coli, the activity of enoyl-CoA hydratase when crotonyl-CoA (4 carbons), 2-hexenoyl-CoA (6 carbons) or 2-octenoyl-CoA (8 carbons) was used as the substrate was determined according to the method described in Non-patent document 4. Thus, the purified enzyme was added to 100 mM Tris-HCl buffer (pH 7.8) containing the substrate (a total volume of 400 µl), and reduction in absorbance at 263 nm was measured with a spectrophotometer. At this time, the measured values obtained by varying the substrate concentration were analyzed by the Lineweaver-Burk plot to calculate the Michaelis constant Km, and the maximum velocity Vmax. The result is shown in Table 4. The result revealed that any of PhaJ1, PhaJ2 and PhaJ3 is enoyl-CoA hydratase that exhibits a higher catalytic efficiency as the substrate chain length increases.

### [Table 4]

**Table 4. The Michaelis constant Km and the maximum velocity Vmax in the enoyl-CoA hydratase activity of PhaJ1, PhaJ2 and PhaJ3 when 2-enoyl-CoA having a carbon chain length of 4-8 was used as the substrate**

| Enzym e | Substrate | Michaelis constant Kₘ (µM) | Maximum velocity Vmax (U mg⁻¹) | Catalytic efficiency Vmax/Km (U mg⁻¹M⁻¹) |
|---|---|---|---|---|
| PhaJ1 | Crotonyl-CoA (C4) | 97±5 | 8.2±0.2×10¹ | 8.5×150 |
| | 2-hexenoyl-CoA (C6) | 41±7 | 7.0±0.5×10² | 1.7×10⁷ |
| | 2-oxtenoyl-CoA (C8) | 24±5 | 6.7±0.7×10² | 2.8×10⁷ |
| PhaJ2 | Crotonyl-CoA (C4) | 311±15 | 1.4±0.1×10² | 4.5×10⁵ |
| | 2-hexenoyl-CoA (C6) | 77±14 | 2.4±0.3×10³ | 3.1×10⁷ |
| | 2-oxtenoyl-CoA (C8) | 23±4 | 2.0±0.1×10³ | 8.7×10⁷ |
| PhaJ3 | Crotonyl-CoA (C4) | 178±46 | 1.3±0.2×10¹ | 7.3×10⁴ |
| | 2-hexenoyl-CoA (C6) | 110±16 | 1.1±0.1×10² | 1.0×10⁵ |
| | 2-oxtenoyl-CoA (C8) | 38±12 | 2.8±0.5×10² | 7.4×10⁶ |

In the above measurement of enoyl-CoA hydratase activity, no findings can be obtained on stereoselectivity at the addition of water to the double bond. Thus, the stereoselectivity of the hydration reaction by PhaJ1, PhaJ2 and PhaJ3 was investigated as follows. From the cells of C. necator that accumulated PHA, a water-insoluble PHA granule fraction can be prepared, but PHA synthetic synthase is bound to the surface of the PHA granules. This PHA synthase specifically polymerizes (R)-3-hydroxyacyl-CoA and releases CoA-SH. Thus, when both of the PHA granule fraction and 5,5'-dithiobis(2-nitrobenzoic acid) (DTNB) are present in the reaction solution for determining enoyl-CoA hydratase activity, COA-SH released together with the polymerization by PHA synthase reacts with DTNB to produce a yellow dye having a specific absorption at 412 nm, if the specificity of enoyl-CoA hydratase is (R)-form-specific. Conversely, when enoyl-CoA hydratase is (S)-form-specific, no yellow dye is produced.

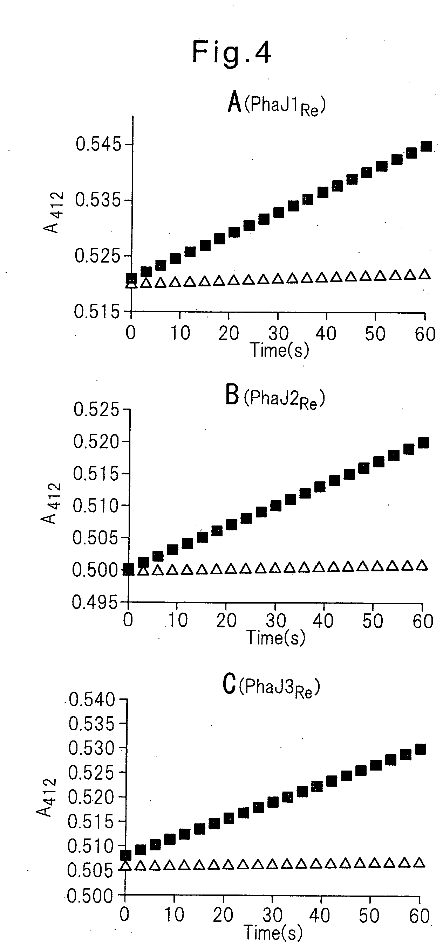

Measurement was carried out according to the method described in Non-patent document 4. Thus, the PHA granule fraction was prepared from the cells of the C. necator wild type strain (the H16 strain) cultured for 36 hours in 100 ml of a medium containing fructose as a carbon source in a method described in Example 18. The cells were collected by centrifugation from the culture, and suspended in 3 ml of 50 mM Tris-HCl buffer (pH 7.5) followed by sonic-disruption. The supernatant obtained by low-speed centrifugation of the disruption liquid at 1,500xg, 5 min was further subjected to high-speed centrifugation at 15,000×g, 15 min. And the precipitate fraction was suspended in 3 ml of 50 mM Tris-HCl buffer (pH 7.5) to prepare a PHA granule fraction. Then, the purified enzyme was added to 100 mM Tris-HCl buffer (pH 7.8) containing 0.2 mM crotonyl-CoA, 1 mM DTNB and 5 µl PHA granule fraction (a total volume of 400 µl), and an increase in absorbance at 412 nm was measured. As a result, as shown in Fig. 4, when PhaJ1, PhaJ2 and PhaJ3 were added, an increase in absorbance at 412 nm was observed. This result revealed that all of PhaJ1, PhaJ2 and PhaJ3 are (R)-form-specific enoyl-CoA hydratases.

## Claims

1. A method of producing poly(3-hydroxybutyrate-co-3-hydroxyhexanoate), which method comprises transforming, by homologous recombination, the (R)-form-specific enoyl CoA hydratase gene into the chromosome of a recombinant Cupriavidus necator strain to which a poly(3-hydroxybutyrate-co-3-hydroxyhexanoate)-producing ability has been conferred, or transforming by introducing into said strain an autonomous replicating vector having said gene integrated therein, and growing the transformant in a medium containing a vegetable oil as a carbon source, wherein the fraction of 3-hydroxyhexanoate is 5-20 mol% and the accumulation rate of poly(3-hydroxybutyrate-co-3-hydroxyhexanoate) in the transformant is 50-90% by weight.

2. The method according to claim 1 wherein said recombinant Cupriavidus necator strain is a NSDG strain, a NSDGAA strain, or a MF01 strain.

3. The method according to claim 1 or 2, wherein the (R)-form-specific enoyl CoA hydratase gene:
is derived from an Aeromonas caviae strain, and comprises
(a) a nucleic acid comprising the base sequence set forth in SEQ ID NO: 1, or
(b) a nucleic acid that hybridizes to a nucleic acid comprising the base sequence set forth in SEQ ID NO: 1 under a stringent condition and that encodes a protein having an activity of converting a fatty acid β-oxidation system intermediate to (R)-3-hydroxyacyl-CoA.

4. The method according to claim 1 or 2, wherein the R form-specific enoyl CoA hydratase gene:
is derived from a Cupriavidus necator strain, and comprises
(a) a nucleic acid comprising the base sequence set forth in SEQ ID NO: 2, or
(b) a nucleic acid that hybridizes to a nucleic acid comprising the base sequence set forth in SEQ ID NO: 2 under a stringent condition and that encodes a protein having an activity of converting a fatty acid β-oxidation system intermediate to (R)-3-hydroxyacyl-CoA.

5. The method according to claim 1 or 2, wherein the (R)-form-specific enoyl CoA hydratase gene:
is derived from a Cupriavidus necator strain, and comprises
(a) a nucleic acid comprising the base sequence set forth in SEQ ID NO: 3, or
(b) a nucleic acid that hybridizes to a nucleic acid comprising the base sequence set forth in SEQ ID NO: 3 under a stringent condition and that encodes a protein having an activity of converting a fatty acid β-oxidation system intermediate to (R)-3-hydroxyacyl-CoA.

6. The method according to claim 1 or 2, wherein the R form-specific enoyl CoA hydratase gene:
- is derived from an Aeromonas caviae strain, and comprises
(a) a nucleic acid comprising the base sequence set forth in SEQ ID NO: 1, or
(b) a nucleic acid that hybridizes to a nucleic acid comprising the base sequence set forth in SEQ ID NO: 1 under a stringent condition and that encodes a protein having an activity of converting a fatty acid β-oxidation system intermediate to (R)-3-hydroxyacyl-CoA; and
- is derived from a Cupriavidus necator strain, and comprises
(a) a nucleic acid comprising the base sequence set forth in SEQ ID NO: 2, or
(b) a nucleic acid that hybridizes to a nucleic acid comprising the base sequence set forth in SEQ ID NO: 2 under a stringent condition and that encodes a protein having an activity of converting a fatty acid β-oxidation system intermediate to (R)-3-hydroxyacyl-CoA.

7. The method according to claim 1 or 2, wherein the R form-specific enoyl CoA hydratase gene:
- is derived from an Aeromonas caviae strain, and comprises
(a) a nucleic acid comprising the base sequence set forth in SEQ ID NO: 1, or
(b) a nucleic acid that hybridizes to a nucleic acid comprising the base sequence set forth in SEQ ID NO: 1 under a stringent condition and that encodes a protein having an activity of converting a fatty acid β-oxidation system intermediate to (R)-3-hydroxyacyl-CoA; and
- is derived from a Cupriavidus necator strain, and comprises
(a) a nucleic acid comprising the base sequence set forth in SEQ ID NO: 3, or
(b) a nucleic acid that hybridizes to a nucleic acid comprising the base sequence set forth in SEQ ID NO: 3 under a stringent condition and that encodes a protein having an activity of converting a fatty acid β-oxidation system intermediate to (R)-3-hydroxyacyl-CoA.

## Patentansprüche

1. Verfahren zur Herstellung von Poly(3-hydroxybutyrat-co-3-hydroxyhexanoat), wobei das Verfahren das Transformieren des (R)-Form-spezifischen Enoyl-CoA-Hydratase-Gens durch homologe Rekombination in das Chromosom eines rekombinanten Cupriavidus necator-Stamms, dem eine Poly(3-hydroxybutyrat-co-3-hydroxyhexanoat)-produzierende Fähigkeit verliehen wurde oder Transformieren durch Einführen eines autonom replizierenden Vektors, in den das Gen integriert wurde, in den Stamm, und Anzucht der Transformante in einem Medium, das ein Pflanzenöl als Kohlenstoffquelle enthielt, umfasst, wobei die 3-Hydroxyhexanoat-Fraktion 5-20 Mol-% beträgt und die Akkumulationsrate von Poly(3-hydroxybutyrat-co-3-hydroxyhexanoat) in der Transformante 50-90 Gew.-% beträgt.

2. Verfahren nach Anspruch 1, wobei der rekombinante Cupriavidus necator-Stamm ein NSDG-Stamm, ein NSDGΔA-Stamm oder ein MF01-Stamm ist.

3. Verfahren nach Anspruch 1 oder 2, wobei das (R)-Form-spezifische Enoyl-CoA-Hydratase-Gen:
von einem Aeromonas caviae-Stamm abgeleitet ist und es umfasst
(a) eine Nukleinsäure, die die in SEQ ID NO: 1 angegebene Basensequenz umfasst oder
(b) eine Nukleinsäure, die unter stringenter Bedingung an eine Nukleinsäure hybridisiert, die die in SEQ ID Nr. 1 angegebene Basensequenz umfasst, und die ein Protein mit einer Aktivität zur Umwandlung eines Zwischenprodukts des Fettsäure-β-Oxidationssystems zu (R)-3-Hydroxyacyl-CoA codiert.

4. Verfahren nach Anspruch 1 oder 2, wobei das R-Formspezifische Enoyl-CoA-Hydratase-Gen:
von einem Cupriavidus necator-Stamm abgeleitet ist und es umfasst:
(a) eine Nukleinsäure, die die in SEQ ID NO: 2 angegebene Basensequenz umfasst, oder
(b) eine Nukleinsäure, die unter stringenter Bedingung an eine Nukleinsäure hybridisiert, die die in SEQ ID NO: 2 angegebene Basensequenz umfasst und die ein Protein mit einer Aktivität zur Umwandlung eines Zwischenprodukts des Fettsäure-β-Oxidationssystems zu (R)-3-Hydroxyacyl-CoA codiert.

5. Verfahren nach Anspruch 1 oder 2, wobei das (R)-Form-spezifische Enoyl-CoA-Hydratase-Gen:
von einem Cupriavidus necator-Stamm abgeleitet ist und es umfasst
(a) eine Nukleinsäure, die die in SEQ ID NO: 3 angegebene Basensequenz umfasst, oder
(b) eine Nukleinsäure, die unter stringenter Bedingung an eine Nukleinsäure hybridisiert, die die in SEQ ID Nr. 3 angegebene Basensequenz umfasst und die ein Protein mit einer Aktivität zur Umwandlung eines Zwischenprodukts des Fettsäure-β-Oxidationssystems zu (R)-3-Hydroxyacyl-CoA codiert.

6. Verfahren nach Anspruch 1 oder 2, wobei das R-Formspezifische Enoyl-CoA-Hydratase-Gen:
- von einem Aeromonas caviae-Stamm abgeleitet ist und es umfasst
(a) eine Nukleinsäure, die die in SEQ ID NO: 1 angegebene Basensequenz umfasst, oder
(b) eine Nukleinsäure, die unter stringenter Bedingung an eine Nukleinsäure hybridisiert, die die in SEQ ID Nr. 1 angegebene Basensequenz umfasst und die ein Protein mit einer Aktivität zur Umwandlung eines Zwischenprodukts des Fettsäure-β-Oxidationssystems zu (R)-3-Hydroxyacyl-CoA codiert; und
- von einem Cupriavidus necator-Stamm abgeleitet ist und es umfasst
(a) eine Nukleinsäure, die die in SEQ ID NO: 2 angegebene Basensequenz umfasst, oder
(b) eine Nukleinsäure, die unter stringenter Bedingung an eine Nukleinsäure hybridisiert, die die in SEQ ID NO: 2 angegebene Basensequenz umfasst und die ein Protein mit einer Aktivität zur Umwandlung eines Zwischenprodukts des Fettsäure-β-Oxidationssystems zu (R)-3-Hydroxyacyl-CoA codiert.

7. Verfahren nach Anspruch 1 oder 2, wobei das R-Formspezifische Enoyl-CoA-Hydratase-Gen:
- von einem Aeromonas caviae-Stamm abgeleitet ist und es umfasst
(a) eine Nukleinsäure, die die in SEQ ID NO: 1 angegebene Basensequenz umfasst, oder
(b) eine Nukleinsäure, die unter stringenter Bedingung an eine Nukleinsäure hybridisiert, die die in SEQ ID Nr. 1 angegebene Basensequenz umfasst und die ein Protein mit einer Aktivität zur Umwandlung eines Zwischenprodukts des Fettsäure-β-Oxidationssystems zu (R)-3-Hydroxyacyl-CoA codiert; und
- von einem Cupriavidus necator-Stamm abgeleitet ist und es umfasst
(a) eine Nukleinsäure, die die in SEQ ID NO: 3 angegebene Basensequenz umfasst, oder
(b) eine Nukleinsäure, die unter stringenter Bedingung an eine Nukleinsäure hybridisiert, die die in SEQ ID NO: 3 angegebene Basensequenz umfasst und die ein Protein mit einer Aktivität zur Umwandlung eines Zwischenprodukts des Fettsäure-β-Oxidationssystems zu (R)-3-Hydroxyacyl-CoA codiert.

## Revendications

1. Procédé de production de poly(3-hydroxybutyrate-co-3-hydroxyhexanoate), lequel procédé consiste à transformer, par recombinaison homologue, le gène de l'énoyl-CoA-hydratase spécifique à la forme (R) dans le chromosome d'une souche de Cupriavidus necator recombinante à laquelle une capacité de production de poly(3-hydroxybutyrate-co-3-hydroxyhexanoate) a été conférée, ou transformer en introduisant dans ladite souche un vecteur de réplication autonome ayant ledit gène intégré en son sein, et faire croître le transformant dans un milieu contenant une huile végétale en tant que source de carbone, la fraction de 3-hydroxyhexanoate étant comprise entre 5 et 20 % molaire et le taux d'accumulation de poly(3-hydroxybutyrate-co-3-hydroxyhexanoate) dans le transformant étant compris entre 50 et 90 % en poids.

2. Procédé selon la revendication 1, dans lequel ladite souche de Cupriavidus necator recombinante est une souche NSDG, une souche NSDGΔA ou une souche MF01.

3. Procédé selon la revendication 1 ou 2, dans lequel le gène de l'énoyl-CoA-hydratase spécifique à la forme (R) :
est dérivé d'une souche d'Aeromonas caviae, et comprend
(a) un acide nucléique comprenant la séquence de bases définie dans la SEQ ID N° : 1, ou
(b) un acide nucléique qui s'hybride avec un acide nucléique comprenant la séquence de bases définie dans la SEQ ID N° : 1 sous une condition stricte et qui code pour une protéine ayant une activité de conversion d'un intermédiaire du système de β-oxydation d'acide gras en (R)-3-hydroxyacyl-CoA.

4. Procédé selon la revendication 1 ou 2, dans lequel le gène de l'énoyl-CoA-hydratase spécifique à la forme (R) :
est dérivé d'une souche de Cupriavidus necator, et comprend
(a) un acide nucléique comprenant la séquence de bases définie dans la SEQ ID N° : 2, ou
(b) un acide nucléique qui s'hybride avec un acide nucléique comprenant la séquence de bases définie dans la SEQ ID N° : 2 sous une condition stricte et qui code pour une protéine ayant une activité de conversion d'un intermédiaire du système de β-oxydation d'acide gras en (R)-3-hydroxyacyl-CoA.

5. Procédé selon la revendication 1 ou 2, dans lequel le gène de l'énoyl-CoA-hydratase spécifique à la forme (R) :
est dérivé d'une souche de Cupriavidus necator, et comprend
(a) un acide nucléique comprenant la séquence de bases définie dans la SEQ ID N° : 3, ou
(b) un acide nucléique qui s'hybride avec un acide nucléique comprenant la séquence de bases définie dans la SEQ ID N° : 3 sous une condition stricte et qui code pour une protéine ayant une activité de conversion d'un intermédiaire du système de β-oxydation d'acide gras en (R)-3-hydroxyacyl-CoA.

6. Procédé selon la revendication 1 ou 2, dans lequel le gène de l'énoyl-CoA-hydratase spécifique à la forme (R) :
- est dérivé d'une souche d'Aeromonas caviae, et comprend
(a) un acide nucléique comprenant la séquence de bases définie dans la SEQ ID N° : 1, ou
(b) un acide nucléique qui s'hybride avec un acide nucléique comprenant la séquence de bases définie dans la SEQ ID N° : 1 sous une condition stricte et qui code pour une protéine ayant une activité de conversion d'un intermédiaire du système de β-oxydation d'acide gras en (R)-3-hydroxyacyl-CoA ; et
- est dérivé d'une souche de Cupriavidus necator, et comprend
(a) un acide nucléique comprenant la séquence de bases définie dans la SEQ ID N° : 2, ou
(b) un acide nucléique qui s'hybride avec un acide nucléique comprenant la séquence de bases définie dans la SEQ ID N° : 2 sous une condition stricte et qui code pour une protéine ayant une activité de conversion d'un intermédiaire du système de β-oxydation d'acide gras en (R)-3-hydroxyacyl-CoA.

7. Procédé selon la revendication 1 ou 2, dans lequel le gène de l'énoyl-CoA-hydratase spécifique à la forme (R) :
- est dérivé d'une souche d'Aeromonas caviae, et comprend
(a) un acide nucléique comprenant la séquence de bases définie dans la SEQ ID N° : 1, ou
(b) un acide nucléique qui s'hybride avec un acide nucléique comprenant la séquence de bases définie dans la SEQ ID N° : 1 sous une condition stricte et qui code pour une protéine ayant une activité de conversion d'un intermédiaire du système de β-oxydation d'acide gras en (R)-3-hydroxyacyl-CoA ; et
- est dérivé d'une souche de Cupriavidus necator, et comprend
(a) un acide nucléique comprenant la séquence de bases définie dans la SEQ ID N° : 3, ou
(b) un acide nucléique qui s'hybride avec un acide nucléique comprenant la séquence de bases définie dans la SEQ ID N° : 3 sous une condition stricte et qui code pour une protéine ayant une activité de conversion d'un intermédiaire du système de β-oxydation d'acide gras en (R)-3-hydroxyacyl-CoA.


### SEQUENCE LISTING

<110> Tokyo Institute of Technology
<120> A process for producing poly(hydroxyalkanoic acids) by recombinant microorganisms transformed with enoyl-CoA hydratase gene
<130> Z541-PCT
<150> JP2010-043017
   <151> 2010-02-26
<160> 32
<210> 1
   <211> 405
   <212> DNA
   <213> Aeromonas caviae R-hydratase gene (phaJAc)
<220>
   <221> CDS
   <222> (1)..(402)
<400> 1
   atgagcgcac aatccctgga agtaggccag aaggcccgtc tcagcaagcg gttcggggcg   60
   gcggaggtag ccgccttcgc cgcgctctcg gaggacttca accccctgca cctggacccg  120
   gccttcgccg ccaccacggc gttcgagcgg cccatagtcc acggcatgct gctcgccagc  180
   ctcttctccg ggctgctggg ccagcagttg ccgggcaagg ggagcatcta tctgggtcaa  240
   agcctcagct tcaagctgcc ggtctttgtc ggggacgagg tgacggccga ggtggaggtg  300
   accgcccttc gcgaggacaa gcccatcgcc accctgacca cccgcatctt cacccaaggc  360
   ggcgccctcg ccgtgacggg ggaagccgtg gtcaagctgc cttaa                  405
<210> 2
   <211> 477
   <212> DNA
   <213> Cupriavidus necator R-hydratase gene (phaJ1)
<220>
   <221> CDS
   <222> (1)..(474)
<400> 2
   atgcgtacca tcgcatcgct ggaagaactg gaaggcctgc agggccagga ggtcgcggtc   60
   agcgactgga tcgaggtgac ccagcagcag gtcaaccagt ttgccgatgc caccggcgac  120
   caccagtgga ttcacatcga cgtggagcgc gcgaagaagg agtcgcccta tggcggcccg  180
   atcgcgcacg gcttcctgac gctgtcgctg ctgcccaagt tcatgcacaa cgcgctgcac  240
   atgccgtcga agatcggcgt taactacggc ctgaaccgcg tgcgcttcac ggcacccgtg  300
   ccggtgggca gcaagctgcg cgcgcgcatc aagctgctga aggtcgagcg tctcgatccg  360
   ctgccgaagt cgcccgaact ggttggcgca cagtcgacct gggaagtcac ggtggagcgc  420
   gaaggcagcg accgtccggt ctgtgtcgcc gagtcgatca cgcgccgcta cgggtga     477
<210> 3
   <211> 456
   <212> DNA
   <213> Cupriavidus necator R-hydratase gene (phaJ2)
<220>
   <221> CDS
   <222> (1)..(453)
<400> 3
   atgaagacct acgagaacat cgccgatctc cagccgctgg ttggagaagt catcggcacg   60
   agcgaatggc tggcgctgga ccaggcccgc atcaatacgt tcgccgacgc cacgggcgac  120
   caccagtgga ttcacgtcga cgtcgagcgc gcgaagaacg gcccgttcgg cgcgccgatc  180
   gcccacggtt tcctgacgct gagcctgctg cctgccttta cgcactccgc gtaccggatc  240
   cggaacagca gcacgggcgt caactatggc ctcgacaagg tgcggtttcc cgccccggtt  300
   ccggtcgaca gcctgctgcg tgcgcaattc aagctgatgt cgtacgaggc gctggagaac  360
   ggcggcgccc agttcaaggt cgagatgatg gtggagcggc agggcggcag caagccggtg  420
   tgcatcgcgg aatcgatcct gcggcgcttt ccctga                            456
<210> 4
   <211> 594
   <212> PRT
   <213> Aeromonas caviae phaC
<400> 4
   Met Ser Gln Pro Ser Tyr Gly Pro Leu Phe Glu Ala Leu Ala His Tyr
   1               5                   10                  15
   Asn Asp Lys Leu Leu Ala Met Ala Lys Ala Gln Thr Glu Arg Thr Ala
               20                  25                  30
   Gln Ala Leu Leu Gln Thr Asn Leu Asp Asp Leu Gly Gln Val Leu Glu
           35                  40                  45
   Gln Gly Ser Gln Gln Pro Trp Gln Leu Ile Gln Ala Gln Met Asn Trp
       50                  55                  60
   Trp Gln Asp Gln Leu Lys Leu Met Gln His Thr Leu Leu Lys Ser Ala
   65                  70                  75                  80
   Gly Gln Pro Ser Glu Pro Val Ile Thr Pro Glu Arg Ser Asp Arg Arg
                   85                  90                  95
   Phe Lys Ala Glu Ala Trp Ser Glu Gln Pro Ile Tyr Asp Tyr Leu Lys
               100                 105                 110
   Gln Ser Tyr Leu Leu Thr Ala Arg His Leu Leu Ala Ser Val Asp Ala
           115                 120                 125
   Leu Glu Gly Val Pro Gln Lys Ser Arg Glu Arg Leu Arg Phe Phe Thr
       130                 135                 140
   Arg Gln Tyr Val Asn Ala Met Ala Pro Ser Asn Phe Leu Ala Thr Asn
   145                 150                 155                 160
   Pro Glu Leu Leu Lys Leu Thr Leu Glu Ser Asp Gly Gln Asn Leu Val
                   165                 170                 175
   Arg Gly Leu Ala Leu Leu Ala Glu Asp Leu Glu Arg Ser Ala Asp Gln
               180                 185                 190
   Leu Asn Ile Arg Leu Thr Asp Glu Ser Ala Phe Glu Leu Gly Arg Asp
           195                 200                 205
   Leu Ala Leu Thr Pro Gly Arg Val Val Gln Arg Thr Glu Leu Tyr Glu
       210                 215                 220
   Leu Ile Gln Tyr Ser Pro Thr Thr Glu Thr Val Gly Lys Thr Pro Val
   225                 230                 235                 240
   Leu Ile Val Pro Pro Phe Ile Asn Lys Tyr Tyr Ile Met Asp Met Arg
                   245                 250                 255
   Pro Gln Asn Ser Leu Val Ala Trp Leu Val Ala Gln Gly Gln Thr Val
               260                 265                 270
   Phe Met Ile Ser Trp Arg Asn Pro Gly Val Ala Gln Ala Gln Ile Asp
           275                 280                 285
   Leu Asp Asp Tyr Val Val Asp Gly Val Ile Ala Ala Leu Asp Gly Val
       290                 295                 300
   Glu Ala Ala Thr Gly Glu Arg Glu Val His Gly Ile Gly Tyr Cys Ile
   305                 310                 315                 320
   Gly Gly Thr Ala Leu Ser Leu Ala Met Gly Trp Leu Ala Ala Arg Arg
                   325                 330                 335
   Gln Lys Gln Arg Val Arg Thr Ala Thr Leu Phe Thr Thr Leu Leu Asp
               340                 345                 350
   Phe Ser Gln Pro Gly Glu Leu Gly Ile Phe Ile His Glu Pro Ile Ile
           355                 360                 365
   Ala Ala Leu Glu Ala Gln Asn Glu Ala Lys Gly Ile Met Asp Gly Arg
       370                 375                 380
   Gln Leu Ala Val Ser Phe Ser Leu Leu Arg Glu Asn Ser Leu Tyr Trp
   385                 390                 395                 400
   Asn Tyr Tyr Ile Asp Ser Tyr Leu Lys Gly Gln Ser Pro Val Ala Phe
                   405                 410                 415
   Asp Leu Leu His Trp Asn Ser Asp Ser Thr Asn Val Ala Gly Lys Thr
               420                 425                 430
   His Asn Ser Leu Leu Arg Arg Leu Tyr Leu Glu Asn Gln Leu Val Lys
           435                 440                 445
   Gly Glu Leu Lys Ile Arg Asn Thr Arg Ile Asp Leu Gly Lys Val Lys
       450                 455                 460
   Thr Pro Val Leu Leu Val Ser Ala Val Asp Asp His Ile Ala Leu Trp
   465                 470                 475                 480
   Gln Gly Thr Trp Gln Gly Met Lys Leu Phe Gly Gly Glu Gln Arg Phe
                   485                 490                 495
   Leu Leu Ala Glu Ser Gly His Ile Ala Gly Ile Ile Asn Pro Pro Ala
               500                 505                 510
   Ala Asn Lys Tyr Gly Phe Trp His Asn Gly Ala Glu Ala Glu Ser Pro
           515                 520                 525
   Glu Ser Trp Leu Ala Gly Ala Thr His Gln Gly Gly Ser Trp Trp Pro
       530                 535                 540
   Glu Met Met Gly Phe Ile Gln Asn Arg Asp Glu Gly Ser Glu Pro Val
   545                 550                 555                 560
   Pro Ala Arg Val Pro Glu Glu Gly Leu Ala Pro Ala Pro Gly His Tyr
                   565                 570                 575
   Val Lys Val Arg Leu Asn Pro Val Phe Ala Cys Pro Thr Glu Glu Asp
               580                 585                 590
   Ala Ala
<210> 5
   <211> 56
   <212> DNA
   <213> Artificial Sequence
<220>
   <223> Oligonucleotide primer used in PCR for amplifying phaCNSPG gene
<400> 5
   ggttcgaata gtgacggcag agagacaatc aaatcatgag ccaaccatct tatggc 56
<210> 6
   <211> 57
   <212> DNA
   <213> Artificial Sequence
<220>
   <223> Oligonucleotide primer used in PCR for amplifying phaCNSPG gene
<400> 6
   aacctgcagg cctgccggcg ccgtgcatat gcaagcgtca tgcggcgtcc tcctctg 57
<210> 7
   <211> 20
   <212> DNA
   <213> Artificial Sequence
<220>
   <223> Oligonucleotide primer used in PCR for amplifying 1041bp gene region including phaR gene
<400> 7
   tcgaccggcg ccgacttctc 20
<210> 8
   <211> 21
   <212> DNA
   <213> Artificial Sequence
<220>
   <223> Oligonucleotide primer used in PCR for amplifying 1041bp gene region including phaR gene
<400> 8
   gcatgccagt gtcttacttc t 21
<210> 9
   <211> 20
   <212> DNA
   <213> Artificial Sequence
<220>
   <223> Oligonucleotide primer used in PCR for amplifying gene region including phaA gene
<400> 9
   cgccgcatga cgcttgcata 20
<210> 10
   <211> 24
   <212> DNA
   <213> Artificial Sequence
<220>
   <223> Oligonucleotide primer used in PCR for amplifying gene region including phaA gene
<400> 10
   ccatatgcgg ccccggaaaa cccc 24
<210> 11
   <211> 19
   <212> DNA
   <213> Artificial Sequence
<220>
   <223> Oligonucleotide primer used in PCR for amplifying gene region including phaB1 gene
<400> 11
   cgcatatggt tggcgcgga 19
<210> 12
   <211> 31
   <212> DNA
   <213> Artificial Sequence
<220>
   <223> Oligonucleotide primer used in PCR for amplifying gene region including phaB1 gene
<400> 12
   gcccggatcc tatgcccaac aaggcactaa g 31
<210> 13
   <211> 27
   <212> DNA
   <213> Artificial Sequence
<220>
   <223> Oligonucleotide primer used in PCR for amplifying 1kbp gene region upstream of phaA
<400> 13
   tgcgaattca gaactccctg gtcgcct 27
<210> 14
   <211> 45
   <212> DNA
   <213> Artificial Sequence
<220>
   <223> Oligonucleotide primer used in PCR for amplifying 1kbp gene region upstream of phaA
<400> 14
   cgtcggatcc tctagattcg aagcgtcatg cggcgtcctc ctctg 45
<210> 15
   <211> 45
   <212> DNA
   <213> Artificial Sequence
<220>
   <223> Oligonucleotide primer used in PCR for amplifying 1kbp gene region downstream of phaA
<400> 15
   acgcttcgaa tctagaggat ccgacgataa cgaagccaat caagg 45
<210> 16
   <211> 37
   <212> DNA
   <213> Artificial Sequence
<220>
   <223> Oligonucleotide primer used in PCR for amplifying 1kbp gene region downstream of phaA
<400> 16
   tgcgcaagct ttatgcccaa caaggcacta agaaaag 37
<210> 17
   <211> 64
   <212> DNA
   <213> Artificial Sequence
<220>
   <223> Oligonucleotide primer used in PCR for amplifying Aeromonas caviae R-hydratase gene (phaJAC)
<400> 17
   catctcctgc aggttcgaag aggaggacgc cgcatgagcg cacaatccct ggaagtaggc 60
   caga 64
<210> 18
   <211> 43
   <212> DNA
   <213> Artificial Sequence
<220>
   <223> Oligonucleotide primer used in PCR for amplifying Aeromonas caviae R-hydratase gene (phaJAC)
<400> 18
   cgccacctgc aggctctaga ttaaggcagc ttgaccacgg ctt 43
<210> 19
   <211> 49
   <212> DNA
   <213> Artificial Sequence
<220>
   <223> Oligonucleotide primer used in PCR for amplifying btkB gene region
<400> 19
   tacattctag aaaggaggca aagtcatgac gcgtgaagtg gtagtggta 49
<210> 20
   <211> 43
   <212> DNA
   <213> Artificial Sequence
<220>
   <223> Oligonucleotide primer used in PCR for amplifying btkB gene region
<400> 20
   gctcaggatc caccccttcc tcagatacgc tcgaagatgg cgg 43
<210> 21
   <211> 40
   <212> DNA
   <213> Artificial Sequence
<220>
   <223> Oligonucleotide primer used in PCR for amplifying phaP promoter region
<400> 21
   aaagatctaa tattgttctg ttcacgtctt tgttagttcc 40
<210> 22
   <211> 63
   <212> DNA
   <213> Artificial Sequence
<220>
   <223> Oligonucleotide primer used in PCR for amplifying phaP promoter region
<400> 22
   gaggatcccc gggtacctat gaattcatat gtatatacct cccaggcgtg tgggtgggtc 60
   aag 63
<210> 23
   <211> 26
   <212> DNA
   <213> Artificial Sequence
<220>
   <223> Oligonucleotide primer used in PCR for amplifying Cupriavidus Necator R-hydratase gene (phaJ1)
<400> 23
   tccatatgcg taccatcgca tcgctg 26
<210> 24
   <211> 28
   <212> DNA
   <213> Artificial Sequence
<220>
   <223> Oligonucleotide primer used in PCR for amplifying Cupriavidus Necator R-hydratase gene (phaJ1)
<400> 24
   cgctcgagtc acccgtagcg gcgcgtga 28
<210> 25
   <211> 26
   <212> DNA
   <213> Artificial Sequence
<220>
   <223> Oligonucleotide primer used in PCR for amplifying Cupriavidus Necator R-hydratase gene (phaJ2)
<400> 25
   cacatatgaa gacctacgag aacatc 26
<210> 26
   <211> 26
   <212> DNA
   <213> Artificial Sequence
<220>
   <223> Oligonucleotide primer used in PCR for amplifying Cupriavidus Necator R-hydratase gene (phaJ2)
<400> 26
   ctcgagtcag ggaaagcgcc gcagga 26
<210> 27
   <211> 26
   <212> DNA
   <213> Artificial Sequence
<220>
   <223> Oligonucleotide primer used in PCR for amplifying Cupriavidus Necator R-hydratase gene (phaJ3)
<400> 27
   cacatatgcc aagaatcttc cgttct 26
<210> 28
   <211> 27
   <212> DNA
   <213> Artificial Sequence
<220>
   <223> Oligonucleotide primer used in PCR for amplifying Cupriavidus Necator R-hydratase gene (phaJ3)
<400> 28
   atctcgagtc agaagtaata gcggctg 27
<210> 29
   <211> 53
   <212> DNA
   <213> Artificial Sequence
<220>
   <223> Oligonucleotide primer used in PCR for amplifying Cupriavidus Necator R-hydratase gene (phaJ1)
<400> 29
   ttgacactag ttctagaagg aggagtatat acatatgcgt accatcgcat cgc 53
<210> 30
   <211> 46
   <212> DNA
   <213> Artificial Sequence
<220>
   <223> Oligonucleotide primer used in PCR for amplifying Cupriavidus Necator R-hydratase gene (phaJ1)
<400> 30
   gccaagcggc cgcttcgaaa ctagttgcag ctcgactcac ccgtag 46
<210> 31
   <211> 52
   <212> DNA
   <213> Artificial Sequence
<220>
   <223> Oligonucleotide primer used in PCR for amplifying Cupriavidus Necator R-hydratase gene (phaJ2)
<400> 31
   gacccactag ttctagaagg aggaatatac atatgaagac ctacgagaac at 52
<210> 32
   <211> 46
   <212> DNA
   <213> Artificial Sequence
<220>
   <223> Oligonucleotide primer used in PCR for amplifying Cupriavidus Necator R-hydratase gene (phaJ2)
<400> 32
   caagcgcggc cgcttcgaaa ctagttcagg gaaagcgccg caggat 46